# EUROPEAN PATENT APPLICATION

(11) **EP 2 085 380 A1**
(43) Date of publication of application: **05.08.2009**
(21) Application number: 07831047.1
(22) Date of filing: 01.11.2007
(51) Int. Cl.: C07C 401/00, A61K 31/59

(54) **PROCESS FOR PRODUCTION OF 1-HYDROXY-19-NORCYCLOVITAMIN D DERIVATIVE AND INTERMEDIATE FOR THE PRODUCTION**

(30) Priority: 02.11.2006 JP 2006298594
(71) Applicant: MERCIAN CORPORATION, Chuo-ku, Tokyo 104-8305 (JP)
(72) Inventor: TOYODA, Asako, Iwata-shi Shizuoka 4380078 (JP); NAGAI, Hazuki, Iwata-shi Shizuoka 4380078 (JP); KONUKI, Kaname, Iwata-shi Shizuoka 4380078 (JP); TSUCHIDA, Toshio, Shizuoka 4380077 (JP)
(74) Representative: Patentanwälte Ruff, Wilhelm, Beier, Dauster & Partner
(86) International application number: PCT/JP2007/071313
(87) International publication number: WO 2008/053961

(57) **Abstract**

A cyclovitamin D derivative produced from 25-hydroxyvitamin D is reacted with osmium tetraoxide or a permanganic acid salt to produce a 10,19-diolcyclovitamin D derivative. The 10,19-diolcyclovitamin D derivative is reacted with a perhalogenic acid salt or lead tetraacetate to produce a 10-oxocyclovitamin D derivative. A 1-hydroxycyclovitamin D derivative is produced from the 10-oxocyclovitamin D derivative via a cyclovitamin D derivative and a 1,10-olefincyclovitamin D derivative. The 1-hydrocyclovitamin D derivative is subjected to solvolysis, thereby producing a 1-hydroxy-19-norviatmin D derivative. Thus provided are a novel process for production of 1-hydroxy-19-norcyclovitamin D derivative that is utilizable as an intermediate in the synthesis of 1-hydroxy-19-norvitamin D derivative which is useful as a pharmaceutical agent; and an intermediate for the production.

## Description

### Cross-reference to Related Applications

The present application claims priority under Japanese Patent Application No. 2006-298594, filed on November 2, 2006, the entire contents of which are hereby incorporated by reference.

### [Technical Field]

The present invention relates to a process for production of 1-hydroxy-19-norcyclovitamin D derivatives that are utilizable as intermediates in the synthesis of vitamin D derivatives that are useful as medicaments.

### [Background Art]

1-Hydroxy-19-norvitamin D derivatives are a group of compounds known to exhibit various biological activities. Among them, 25-hydroxyvitamin D₂ derivates in the form of paricalcitol having formula (A) below have been discovered to exhibit differentiation-inducing activity on malignant cells (see Patent Document 1, Nonpatent Documents 1 and 2). They are widely employed in the treatment of hyperthyroidism in chronic renal failure patient. One known process of producing 25-hydroxy-vitamin D derivatives of the 1-hydroxy-19-nor type, which includes paricalcitol, is a process employing 25-hydroxyvitamin D as a starting material (see Patent Document 1). However, this process is not highly efficient method.

Another process is that of reacting an indene derivative with a phosphine oxide derivative (see Patent Document 2). However, the problem of producing the starting materials remains. Numerous processes of producing the indene derivative employed by this process are known (see Patent Document 3 and Nonpatent Documents 3 to 7). However, all of these processes employ a chemical process such as ozone decomposition to sever the double bonds at positions 7 and 8 and positions 22 and 23 in vitamin D₂, and involve introducing the side chain of VD₂ (position 23 and beyond) hydroxylated at position 25, which is separately synthesized in multiple steps, onto the indene derivative from which the side chain has been cut away, or employ an extremely large number of steps to hydroxylate position 25 on the indene derivative from which the side chain has been cut away. None of these processes is satisfactory. Thus, there is great need for an efficient process of producing 25-hydroxyvitamin D derivatives of the 1-hydroxy-19-nor type.

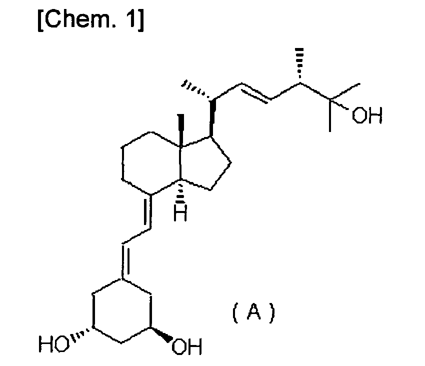

[Patent Document 1] Published Japanese Translation (TOKUHYO) Heisei No. 3-505330 of a PCT International Application, or See the English family member thereof: WO 90/10620.
[Patent Document 2] Japanese Unexamined Patent Publication (KOKAI) Heisei No. 5-221960, or See English family members thereof: USP 5,281,731 and 5,391,755.
[Patent Document 3] USP 4,804,502.
[Nonpatent Document 1] J. Org. Chem. 45, 3235 (1980).
[Nonpatent Document 2] Tetrahedron. Lett. 31, 1823 (1990).
[Nonpatent Document 3] J. Org. Chem. 51, 3098 (1986).
[Nonpatent Document 4] J. Org. Chem. 51, 1264 (1986).
[Nonpatent Document 5] J. Chem. Soc. Perkin Trans. 1,834 (1978).
[Nonpatent Document 6] J. Org. Chem. 48, 1414 (1983).
[Nonpatent Document 7] J. Org. Chem. 51, 1269 (1986).

The entire contents of above-cited Patent Documents 1 to 3 and Nonpatent Documents 1 to 7 are hereby incorporated by reference.

### [Disclosure of the Invention]

The present invention provides a novel process for producing the 1-hydroxy-19-norvitamin D derivatives denoted by formula (X), which are utilizable as intermediates in the synthesis of 1-hydroxy-19-norvitamin D derivatives, including paricalcitol, and provides the intermediates that are manufactured by this process.

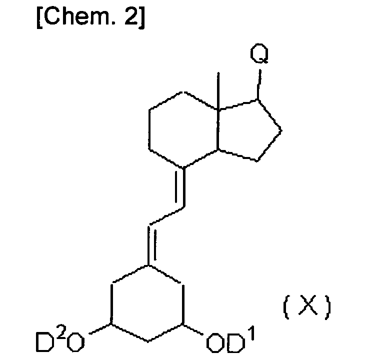

(In the formula, Q denotes a hydrogen atom, optionally substituted C₁ to C₅ alkyl group,

or formula (Q-I) 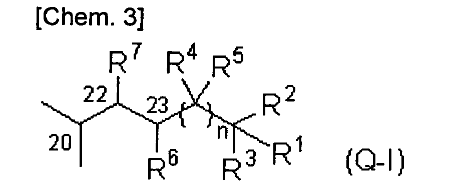 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom),
and each of D¹ and D² denote hydrogen atoms or C₁ to C₇ acyloxy groups.)

The present inventors conducted extensive research into achieving the above-stated object. This resulted in the discovery that by subjecting the cyclovitamin D derivative denoted by formula (IV), which could be synthesized using 25-hydroxy-vitamin D derivative as starting material, to a process comprised of six steps, including an oxidation reaction, it was possible to efficiently manufacture the 1-hydroxy-19-norvitamin D derivative denoted by formula (X) above. The present invention was devised on the basis of this knowledge.

Accordingly, the present invention provides [1] to [25] below:
[1] A process for producing the 1-hydroxy-19-norvitamin D derivative denoted by formula (X): 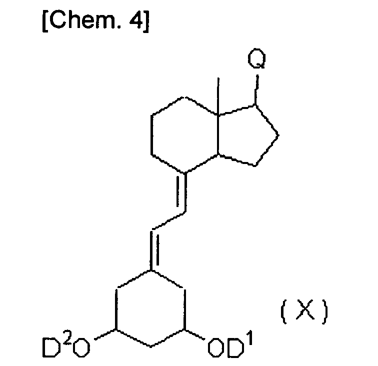 (wherein Q denotes a hydrogen atom, optionally substituted C₁ to C₅ alkyl group

or (Q-I) 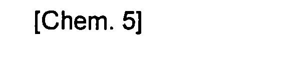 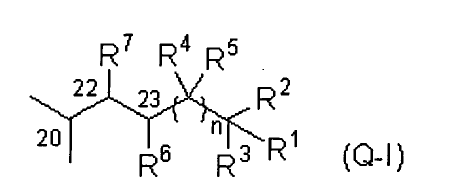 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combined to denote a carbon-carbon double bond; n denotes an integer of 1 to 5; and one of the atoms at position 20, 22 or 23 is optionally substituted with an oxygen, sulfur or nitrogen atom),
and each of D¹ and D² denote hydrogen atoms or C₁ to C₇ acyloxy groups), characterized by:

a) reacting the cyclovitamin D derivative denoted by formula (IV): 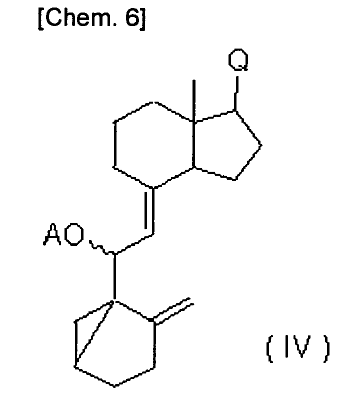 (wherein A denotes a C₁ to C₃ alkyl group and Q is defined as above)
with osmium tetraoxide or a permanganate salt in an inert solvent

to produce the 10,19-diolcyclovitamin D derivative denoted by formula (V): 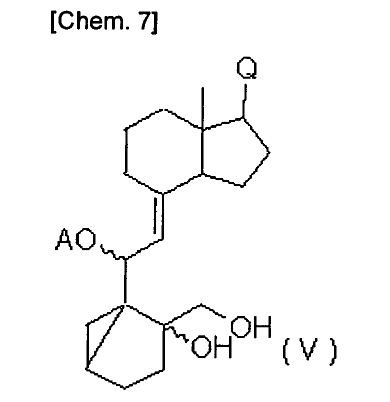 (wherein Q and A are defined as above);

b) reacting the 10,19-diolcyclovitamin D derivative of formula (V) thus obtained with a perhalogenate salt or with lead tetraacetate in an inert solvent to produce the 10-oxocyclovitamin D derivative denoted by general formula (VI): 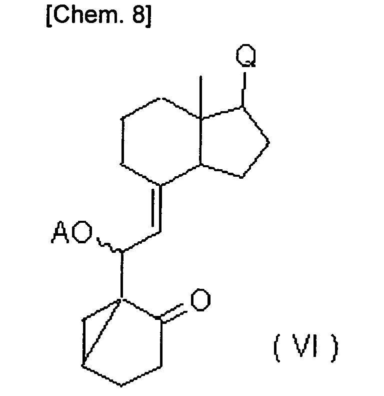 (wherein Q and A are defined as above);

c) reacting the 10-oxocyclovitamin D derivative of formula (VI) thus obtained with PY₅ (wherein Y denotes a halogen atom) or a base and R⁸SO₂-Z (wherein R⁸ denotes an optionally substituted C₁ to C₆ alkyl group, optionally substituted C₂ to C₆ unsaturated alkyl group, optionally substituted C₆ to C₁₄ aryl group or optionally substituted C₇ to C₂₂ aralkyl group; Z denotes a halogen, R⁸SO₂O-(wherein R⁸ is defined as above) or R⁸SO₂(W)N- (wherein R⁸ is defined as above, W denotes an optionally substituted C₁ to C₆ alkyl group, optionally substituted C₂ to C₆ unsaturated alkyl group, optionally substituted C₆ to C₁₄ aryl group or optionally substituted C₇ to C₂₂ aralkyl group)) in an inert solvent

to produce the cyclovitamin D derivative denoted by formula (VII): 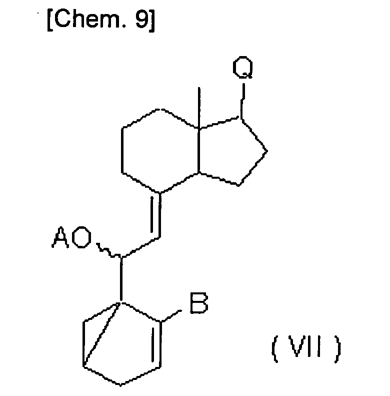 (wherein Q and A are defined as above, B denotes a halogen atom or R⁸SO₂O-(wherein R⁸ is defined as above));

d) reacting the cyclovitamin D derivative of formula (VII) thus obtained with a hydrogen source , an amine and noble metal catalyst in an inert solvent to produce the 1,10-olefincyclovitamin D derivative denoted by formula (VIII): 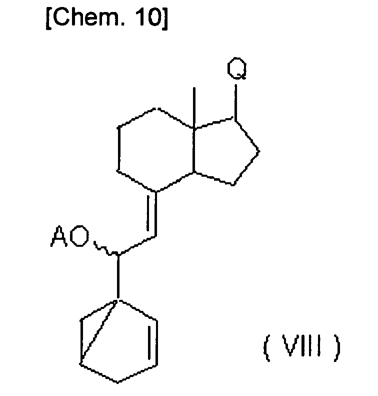 (wherein Q and A are defined as above);

e) reacting the 1,10-olefincyclovitamin D derivative denoted by formula (VIII) thus obtained with a hydroboronating agent in an inert solvent to obtain the 1-hydroxycyclovitamin D derivative denoted by general formula (IX): 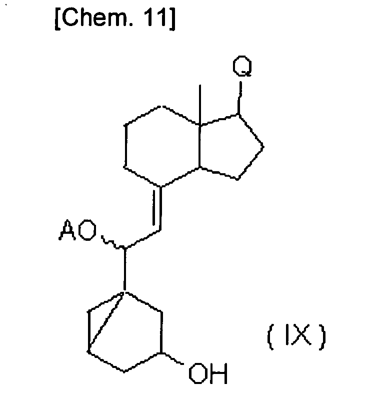 (wherein Q and A are defined as above); and

f) solvolysing the 1-hydroxycyclovitamin D derivative of formula (IX) thus obtained in the presence of an optionally substituted C₁ to C₁₁ organic acid.

[2] A process for producing the cyclovitamin D derivative denoted by formula (VII): 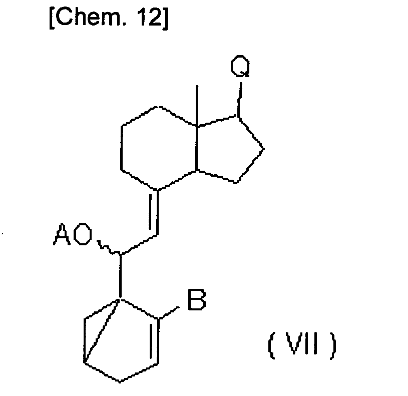 (wherein Q denotes a hydrogen atom, optionally substituted C₁ to C₅ alkyl group, or

(Q-I): 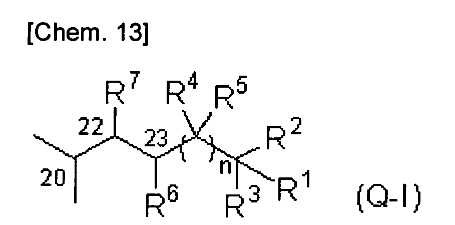 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom),

A denotes a C₁ to C₃ alkyl group, and
B denotes a halogen atom or R⁸SO₂O- (wherein R⁸ denotes an optionally substituted C₁ to C₆ alkyl group, optionally substituted C₂ to C₆ unsaturated alkyl group, optionally substituted C₆ to C₁₄ aryl group or optionally substituted C₇ to C₂₂ aralkyl group)), characterized by:

reacting the 10-oxocyclovitamin D derivative denoted by formula (VI): 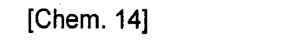 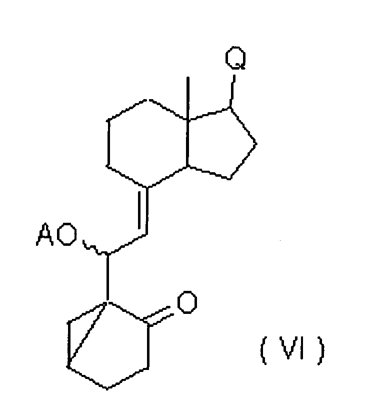 (wherein Q and A are defined as above) with PY₅ (wherein Y denotes a halogen atom) or a base and R⁸SO₂-Z (wherein R⁸ is defined as above, Z denotes a halogen, R⁸SO₂O- (wherein R⁸ is defined as above), or R⁸SO₂(W)N- (wherein R⁸ is defined as above, W denotes an optionally substituted C₁ to C₆ alkyl group, optionally substituted C₂ to C₆ unsaturated alkyl group, optionally substituted C₆ to C₁₄ aryl group, or optionally substituted C₇ to C₂₂ aralkyl group)) in an inert solvent.

[3] The process for producing a cyclovitamin D derivative according to [2], wherein Q denotes formula (Q-I): 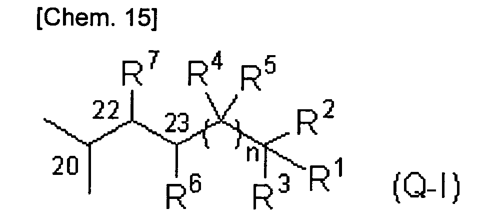 (wherein R¹ denotes a hydrogen atom, hydroxyl group or -OR^{1"}, where R^{1"} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group or propyl group; each of R⁴ and R⁵ denotes a hydrogen atom or methyl group; each of R⁶ and R⁷ denotes a hydrogen atom or R⁶ and R⁷ combine to form a carbon-carbon double bond; n denotes an integer of 1 to 3; and one of the carbons at position 20, 22 or 23 is optionally substituted with an oxygen, sulfur or nitrogen atom);
A denotes a methyl group, and
B denotes R⁸SO₂O- (wherein R⁸ denotes an optionally substituted C₁ to C₄ alkyl group, optionally substituted C₆ or C₇ aryl group, or optionally substituted C₇ or C₈ aralkyl group).

[4] The process for producing a cyclovitamin D derivative according to [2], wherein Q denotes formula (Q-II): 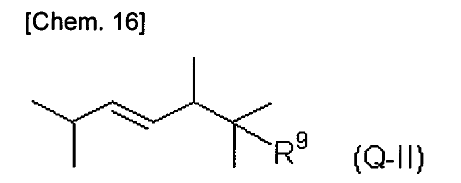 (wherein R⁹ denotes a hydrogen atom, acetoxy group, hydroxyl group, trimethylsilyloxy group, triethylsilyloxy group, methoxymethyloxy group or benzyloxymethyloxy group)

or formula (Q-III) 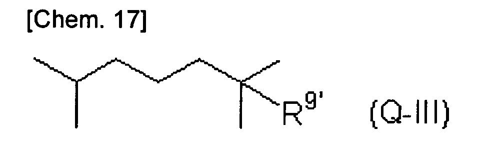 (wherein R^{9'} denotes a hydrogen atom, acetoxy group, trimethylsilyloxy group, triethylsilyloxy group, methoxymethyloxy group or benzyloxymethyloxy group);
A denotes a methyl group and
B denotes a trifluoromethanesulfonyloxy group.

[5] A process for producing a 1,10-olefincyclovitamin D derivative denoted by formula (VIII): 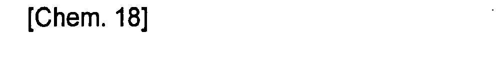 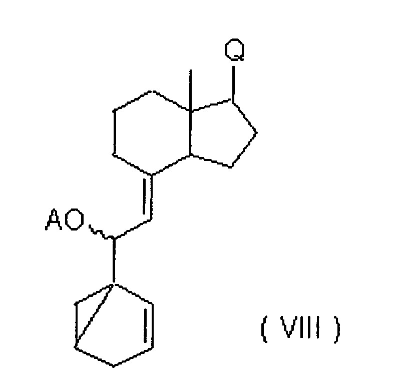 (wherein Q denotes a hydrogen atom, optionally substituted C₁ to C₅ alkyl group or

(Q-I): 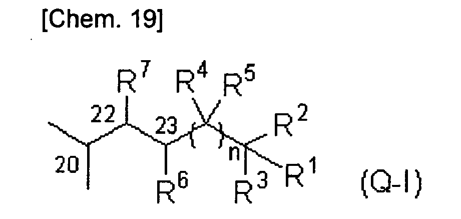 (wherein R¹ denotes a hydrogen atom, hydroxyl group or -OR^{1'}, where R^{1'} denotes optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom)
and A denotes a C₁ to C₃ alkyl group),

characterized by reacting the cyclovitamin D derivative denoted by formula (VII): 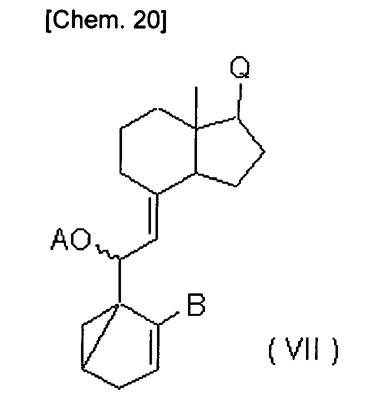 (wherein Q and A are defined as above, B denotes a halogen atom or R⁸SO₂O-(wherein R⁸ denotes an optionally substituted C₁ to C₆ alkyl group, optionally substituted C₂ to C₆ unsaturated alkyl group, optionally substituted C₆ to C₁₄ aryl group or optionally substituted C₇ to C₂₂ aralkyl group)) with an amine, hydrogen source and noble metal catalyst in an inert solvent.

[6] The process for producing a 1,10-olefincyclovitamin D derivative according to [5], wherein Q denotes formula (Q-I): 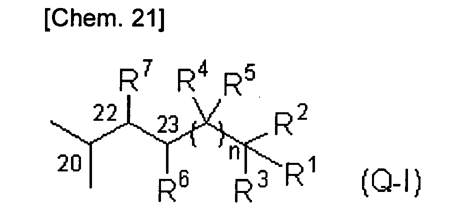 (wherein R¹ denotes a hydrogen atom, hydroxyl group or -OR^{1'}, where R^{1'} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group or propyl group; each of R⁴ and R⁵ denotes a hydrogen atom or methyl group; each of R⁶ and R⁷ denotes a hydrogen atom, or R⁶ and R⁷ combine to form a carbon-carbon double bond; n denotes an integer of 1 to 3; and one of the carbons at position 20, 22 or 23 is optionally substituted with an oxygen, sulfur, or nitrogen atom) and
A denotes a methyl group.

[7] The process for producing a 1,10-olefincyclovitamin D derivative according to [5], wherein Q denotes formula (Q-II): 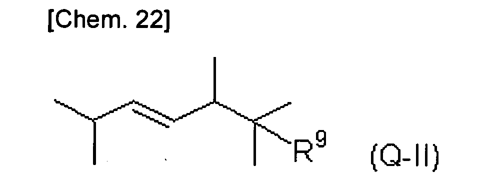 (wherein R⁹ denotes a hydrogen atom, acetoxy group, hydroxyl group, trimethylsilyloxy group, triethylsilyloxy group, methoxymethyloxy group or benzyloxymethyloxy group)

or formula (Q-III): 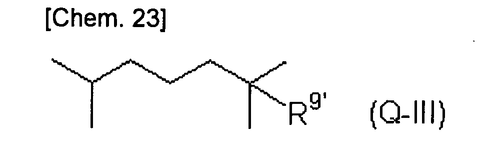 (wherein R^{9'} denotes a hydrogen atom, acetoxy group, trimethylsilyloxy group, triethylsilyloxy group, methoxymethyloxy group, or benzyloxymethyloxy group) and
A denotes a methyl group.

[8] A process for producing a 1-hydroxy-19-norcyclovitamin D derivative denoted by formula (IX): 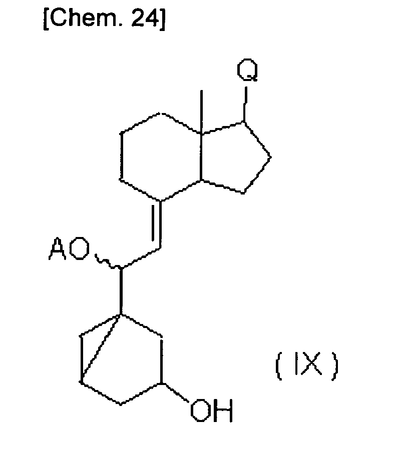 (wherein Q denotes a hydrogen atom, optionally substituted C₁ to C₅ alkyl group,

or (Q-I): 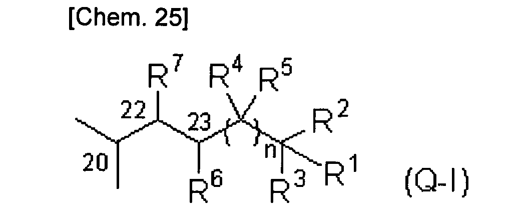 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group, or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom) and
A denotes a C₁ to C₃ alkyl group);

**characterized in that** the 1,10-olefincyclovitamin D derivative denoted by general formula (VIII): 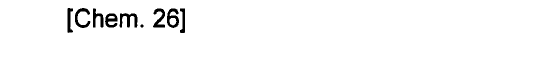 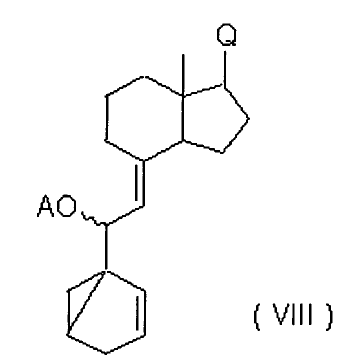 (wherein Q and A are defined as above)
is reacted with a hydroboronating agent in an inert solvent.

[9] The process for producing a 1-hydroxy-19-norchlorovitamin D derivative according to [8], wherein Q denotes formula (Q-I): 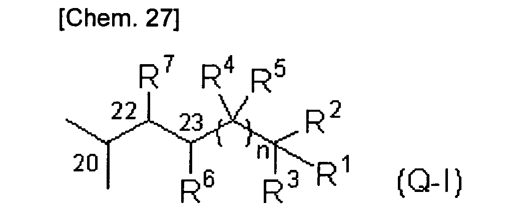 (wherein R¹ denotes a hydrogen atom, hydroxyl group or -OR^{1'}, where R^{1'} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group or propyl group; each of R⁴ and R⁵ denotes a hydrogen atom or methyl group; each of R⁶ and R⁷ denotes a hydrogen atom or R⁶ and R⁷ combine to form a carbon-carbon double bond; n denotes an integer of 1 to 3; and one of the carbons at position 20, 22 or 23 is optionally substituted with an oxygen, sulfur, or nitrogen atom); and
A denotes a methyl group.

[10] The process for producing a 1-hydroxy-19-norchlorovitamin D derivative according to [8], wherein Q denotes formula (Q-II): 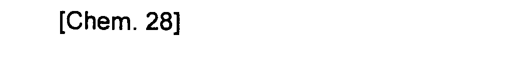 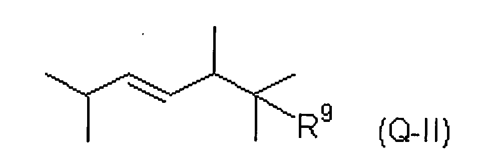 (wherein R⁹ denotes a hydrogen atom, acetoxy group, hydroxyl group, trimethylsilyloxy group, triethylsilyloxy group, methoxymethyloxy group or benzyloxymethyloxy group)

or formula (Q-III): 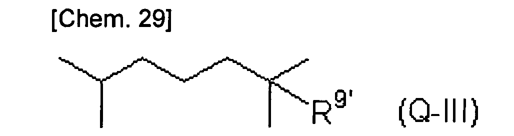 (wherein R^{9'} denotes a hydrogen atom, acetoxy group, trimethylsilyloxy group, triethylsilyloxy group, methoxymethyloxy group or benzyloxymethyloxy group); and
A denotes a methyl group.

[11] The 10-oxocyclovitamin D derivative denoted by formula (VI): 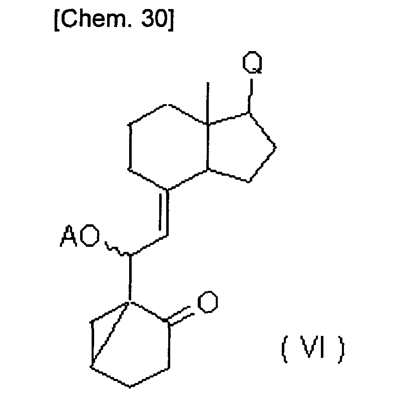 (wherein Q denotes a hydrogen atom, optionally substituted C₁ to C₅ alkyl group

or formula (Q-I): 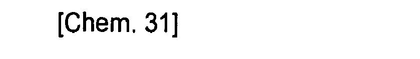 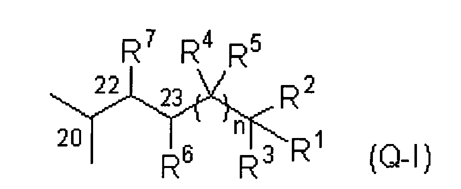 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom);
and A denotes a C₁ to C₃ alkyl group, excluding from the above combinations the case where R¹ denotes a hydroxyl group, R² and R³ denote methyl groups, R⁴, R⁵, R⁶ and R⁷ denote hydrogen atoms, n = 1, and
A denotes a methyl group).

[12] The 10-oxocyclovitamin D derivative of [11], wherein Q denotes formula (Q-I): 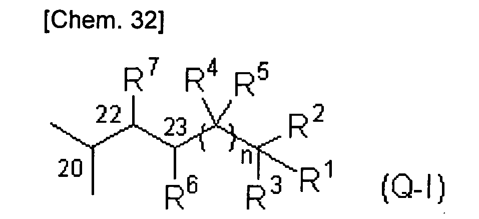 (wherein R¹ denotes a hydrogen atom, hydroxyl group or -OR^{1'}, where R^{1'} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group, or propyl group; each of R⁴ and R⁵ denotes a hydrogen atom or methyl group; each of R⁶ and R⁷ denotes a hydrogen atom, or R⁶ and R⁷ combine to form a carbon-carbon double bond; n denotes an integer of 1 to 3; one of the carbons at position 20, 22 or 23 is optionally substituted with an oxygen, sulfur or nitrogen atom, excluding from the above combinations the case where R¹ denotes a hydroxyl group, R² and R³ denote methyl groups, and R⁴, R⁵, R⁶ and R⁷ denote hydrogen atoms, n = 1) and
A denotes a methyl group.

[13] The 10-oxocyclovitamin D derivative of [11], wherein Q denotes formula (Q-II): 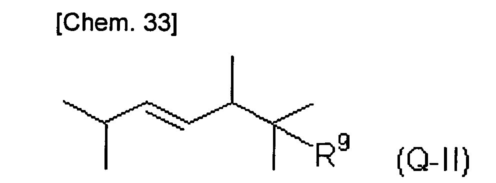 (wherein R⁹ denotes a hydrogen atom, acetoxy group, hydroxyl group, trimethylsilyloxy group, triethylsilyloxy group, methoxymethyloxy group or benzyloxymethyloxy group)

or formula (Q-III): 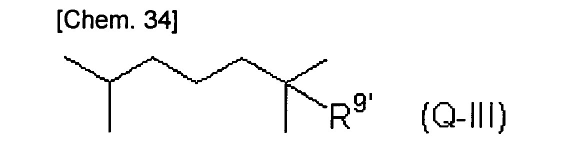 (wherein R^{9'} denotes a hydrogen atom, acetoxy group, trimethylsilyloxy group, triethylsilyloxy group, methoxymethyloxy group or benzyloxymethyloxy group) and
A denotes a methyl group.

[14] The cyclovitamin D derivative denoted by formula (VII): 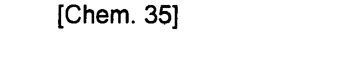 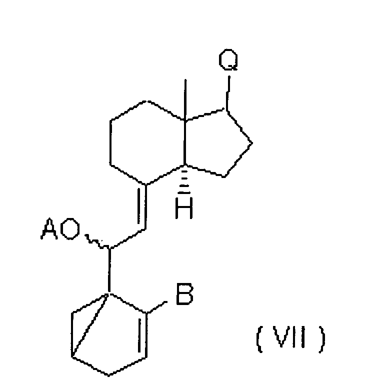 (wherein Q denotes a hydrogen atom, optionally substituted C₁ to C₅ alkyl group,

or formula (Q-I): 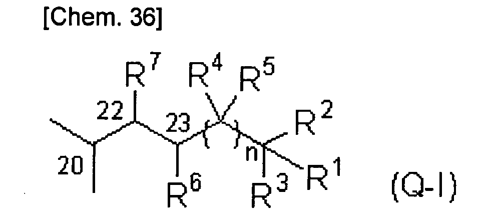 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom);
A denotes a C₁ to C₃ alkyl group; and
B denotes a halogen atom or R⁸SO₂O- (where R⁸ denotes an optionally substituted C₁ to C₆ alkyl group, optionally substituted C₂ to C₆ unsaturated alkyl group, optionally substituted C₆ to C₁₄ aryl group, or optionally substituted C₇ to C₂₂ aralkyl group).

[15] The cyclovitamin D derivative according to [14], wherein Q denotes formula (Q-I): 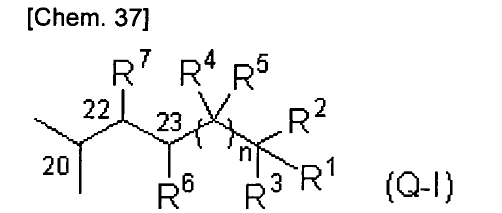 (wherein R¹ denotes a hydrogen atom, hydroxyl group or -OR^{1'}, where R^{1'} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group, or propyl group; each of R⁴ and R⁵ denotes a hydrogen atom or methyl group; each of R⁶ and R⁷ denotes a hydrogen atom, or R⁶ and R⁷ combine to form a carbon-carbon double bond; n denotes an integer of 1 to 3; and one of the carbons at position 20, 22 or 23 is optionally substituted with an oxygen, sulfur or nitrogen atom);
A denotes a methyl group; and
B denotes R⁸SO₂O- (where R⁸ denotes an optionally substituted C₁ to C₄ alkyl group, optionally substituted C₆ or C₇ aryl group or optionally substituted C₇ or C₈ aralkyl group).

[16] The cyclovitamin D derivative according to [14], wherein Q denotes formula (Q-II): 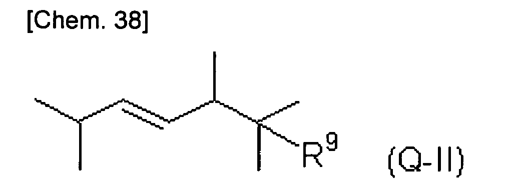 (wherein R⁹ denotes a hydrogen atom, acetoxy group, hydroxyl group, trimethylsilyloxy group, triethylsilyloxy group, methoxymethyloxy group or benzyloxymethyloxy group)

or formula (Q-III): 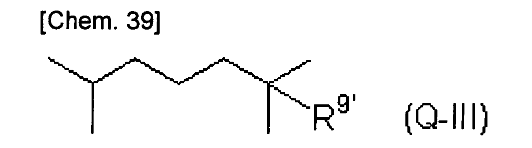 (wherein R^{9'} denotes a hydrogen atom, acetoxy group, trimethylsilyloxy group, triethylsilyloxy group, methoxymethyloxy group or benzyloxymethyloxy group);
A denotes a methyl group; and
B denotes a trifluoromethanesulfonyloxy group.

[17] The 1,10-olefincyclovitamin D derivative denoted by formula (VIII): 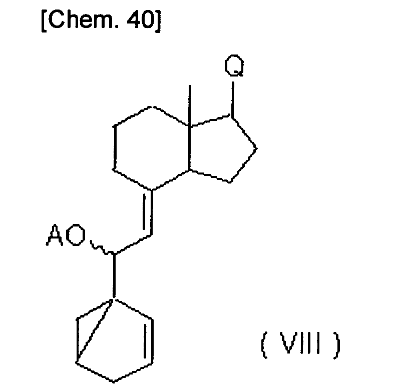 (wherein Q denotes a hydrogen atom, optionally substituted C₁ to C₅ alkyl group,

or formula (Q-I): 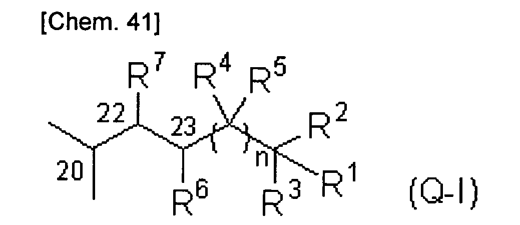 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom); and
A denotes a C₁ to C₃ alkyl group).

[18] The 1,10-olefincyclovitamin D derivative according to [17], wherein Q denotes formula (Q-I): 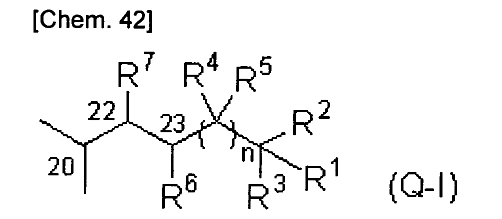 (wherein R¹ denotes a hydrogen atom, hydroxyl group or -OR^{1'}, where R^{1'} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group or propyl group; each of R⁴ and R⁵ denotes a hydrogen atom or methyl group; each of R⁶ and R⁷ denotes a hydrogen atom or R⁶ and R⁷ combine to form a carbon-carbon double bond; n denotes an integer of 1 to 3; and one of the carbons at position 20, 22 or 23 is optionally substituted with an oxygen, sulfur or nitrogen atom); and
A denotes a methyl group.

[19] The 1,10-olefincyclovitamin D derivative according to [17], wherein Q denotes formula (Q-II): 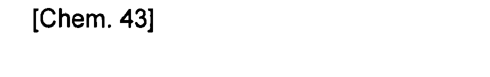 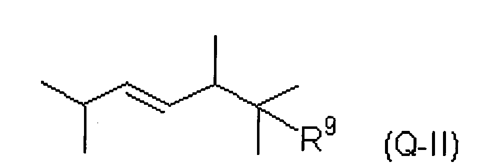 (wherein R⁹ denotes a hydrogen atom, acetoxy group, hydroxyl group, trimethylsilyloxy group, triethylsilyloxy group, methoxymethyloxy group or benzyloxymethyloxy group)

or formula (Q-III): 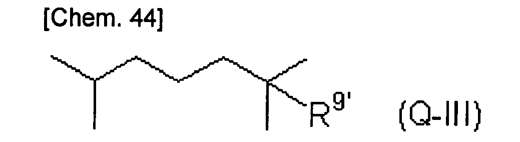 (wherein R^{9'} denotes a hydrogen atom, acetoxy group, trimethylsilyloxy group, triethylsilyloxy group, methoxymethyloxy group or benzyloxymethyloxy group); and A denotes a methyl group.

[20] The 1-hydroxy-19-norcyclovitamin D derivative denoted by formula (IX):

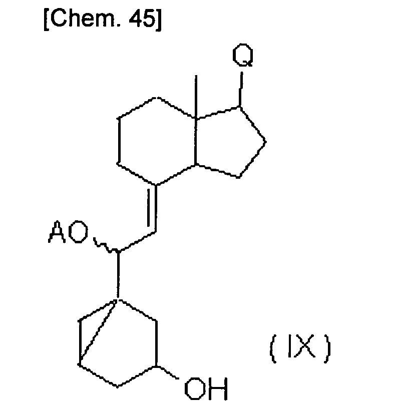

(wherein Q denotes formula (Q-IV): 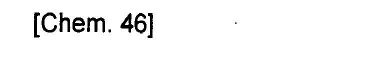 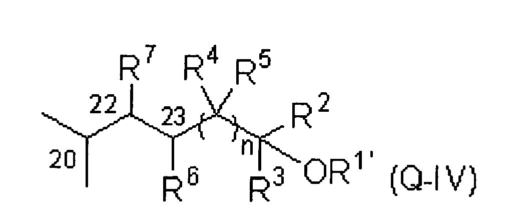 (wherein R^{1'} denotes an optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form -(CH₂)m- (where m denotes an integer of 2 to 5);
each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, or optionally substituted C₂ to C₇ acyloxy group, or R⁶ and R⁷ combine to form a carbon-carbon double bond; n denotes an integer of 1 to 5; and one of the carbon atoms at position 20, 22 or 23 is optionally substituted with an oxygen, sulfur or nitrogen atom); and A denotes a C₁ to C₃ alkyl group.

[21] The 1-hydroxy-19-norcyclovitamin D derivative according to [20], wherein Q denotes formula (Q-IV): 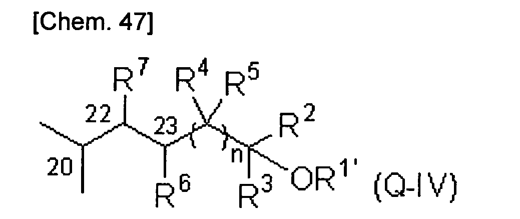 (wherein R^{1'} denotes a trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group or propyl group; each of R⁴ and R⁵ denotes a hydrogen atom or methyl group; each of R⁶ and R⁷ denotes a hydrogen atom, or R⁶ and R⁷ combine to form a carbon-carbon double bond; n denotes an integer of 1 to 3; and one of the carbon atoms at position 20, 22 or 23 is optionally substituted with an oxygen, sulfur or nitrogen atom); and
A denotes a methyl group.

[22] The 1-hydroxy-19-norvitamin D derivative according to [20], wherein Q denotes formula (Q-II): 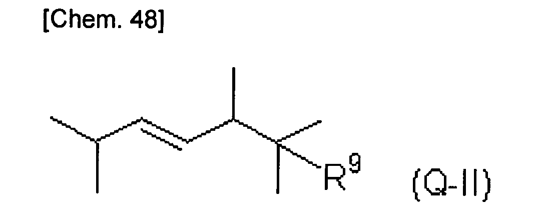 (wherein R⁹ denotes a trimethylsilyloxy group, triethylsilyloxy group, methoxymethyloxy group or benzyloxymethyloxy group)

or formula (Q-III): 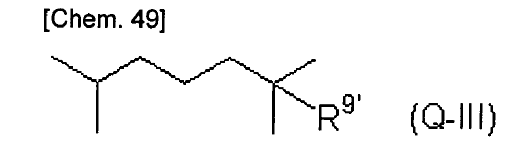 (wherein R^{9'} denotes a trimethylsilyloxy group, triethylsilyloxy group, methoxymethyloxy group or benzyloxymethyloxy group); and
A denotes a methyl group.

[23] The 1-hydroxy-19-norvitamin D derivative denoted by formula (X):

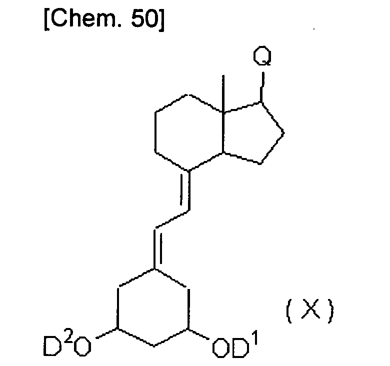

(wherein Q denotes formula (Q-IV): 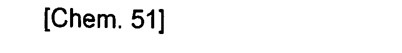 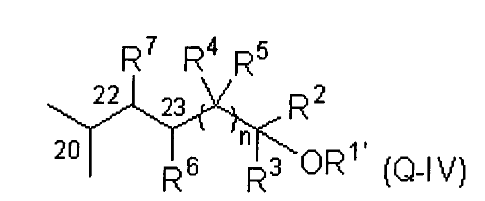 (wherein R^{1'} denotes an optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form -(CH₂)m- (where m denotes an integer of 2 to 5);
each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group or optionally substituted C₂ to C₇ acyloxy group, or R⁶ and R⁷ combine to form a carbon-carbon double bond; n denotes an integer of 1 to 5; and one of the carbon atoms at position 20, 22 or 23 is optionally substituted with an oxygen, sulfur or nitrogen atom); and D¹ and D² denote hydrogen atoms or C₂ to C₇ acyloxy groups.)

[24] The 1-hydroxy-19-norvitamin D derivative according to [23], wherein Q denotes formula (Q-IV): 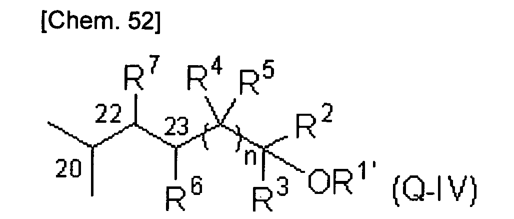 (wherein R^{1'} denotes a trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group or propyl group; each of R⁴ and R⁵ denotes a hydrogen atom or methyl group; each of R⁶ and R⁷ denotes a hydrogen atom, or R⁶ and R⁷ combine to form a carbon-carbon double bond; n denotes an integer of 1 to 3; and one of the carbon atoms at position 20, 22 or 23 is optionally substituted with an oxygen, sulfur or nitrogen atom); and
D¹ and D² denote hydrogen atoms or acetyl groups.

[25] The 1-hydroxy-19-norvitamin D derivative according to [23], wherein Q denotes formula (Q-II): 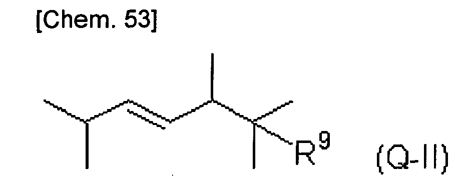 (wherein R⁹ denotes a trimethylsilyloxy group, triethylsilyloxy group, methoxymethyloxy group or benzyloxymethyloxy group)

or formula (Q-III): 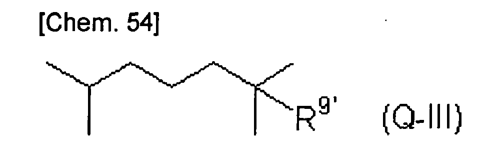 (wherein R^{9'} denotes a trimethylsilyloxy group, triethylsilyloxy group, methoxymethyloxy group or benzyloxymethyloxy group); and
D¹ and D² denotes hydrogen atoms or acetyl groups.

The various terms, symbols, and the like employed in the present specification will be described below.
In the present Specification, the term "halogen atom" means a fluorine, chlorine, bromine, or iodine atom. For example, a fluorine, chlorine, or bromine atom is desirable, and a fluorine or chlorine atom is preferable.

As employed in the specification of the present application, the term "C₁ to C₃ alkyl group" means a linear, branched, or cyclic alkyl group having 1 to 3 carbon atoms, specific examples of which are: methyl, ethyl, n-propyl, isopropyl, and cyclopropyl groups. The term "C₁ to C₄ alkyl group" means a linear, branched, or cyclic alkyl group having 1 to 4 carbon atoms, examples of which are, in addition to the examples given for the "C₁ to C₃ alkyl group" above: n-butyl, isobutyl, sec-butyl, tert-butyl, and cyclobutyl groups. The term "C₁ to C₅ alkyl group" means a linear, branched, or cyclic alkyl group having 1 to 5 carbon atoms, examples of which are, in addition to the examples given for the "C₁ to C₄ alkyl group" above: n-pentyl, 1,1-dimethylpropyl, 1,2-dimethylpropyl, 2,2-dimethylpropyl, 1-ethylpropyl, 1-methylbutyl, 2-methylbutyl, 3-methylbutyl, and cyclopentyl groups.

Similarly, the term "C₁ to C₆ alkyl group" means a linear, branched, or cyclic alkyl group having 1 to 6 carbon atoms, examples of which are, in addition to the examples given for the "C₁ to C₅ alkyl group" above: n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2,3-dimethylbutyl, 1-ethylbutyl, 2-ethylbutyl, 1-methylpentyl, 2-methylpentyl, 3-methylpentyl, 4-methylpentyl, and cyclohexyl groups.

As employed in the specification of the present application, the term "C₂ to C₄ acyl group" means a group on which a carbonyl group is bonded to the terminal of the above-defined "C₁ to C₃ alkyl group." Examples are: acetyl, propionyl, n-butyryl, isobutyryl, and cyclopropanecarbonyl groups.

As employed in the specification of the present application, the term "C₂ to C₇ acyloxy group" means a group on which a carbonyloxy group is bonded to the terminal of the above-defined "C₁ to C₆ alkyl groups." Examples are: acetoxy, propionyloxy, n-butyryloxy, isobutyryloxy, valeryloxy, and isovaleryloxy groups.

As employed in the specification of the present application, the term "C₃ to C₆ alkylsilyl group" means a silyl group having 3 to 6 total carbon atoms that is substituted with one or more alkyl groups. Specific examples are: trimethylsilyl and triethylsilyl groups.

As employed in the specification of the present application, the term "C₂ to C₄ alkoxyalkyl group" means an alkoxyalkyl group having 2 to 4 total carbon atoms. Specific examples are: methoxymethyl, ethoxymethyl, ethoxyethyl, n-propyloxymethyl, and isopropyloxymethyl groups.

As employed in the specification of the present application, the term "C₂ to C₆ unsaturated alkyl group" means an alkyl group having 2 to 6 total carbon atoms, having a double bond. Specific examples are vinyl, allyl, 1-butenyl, 2-butenyl, 3-butenyl, 1-cyclohexenyl, and 2-cyclohexenyl groups.

As employed in the specification of the present application, the term "C₆ or C₇ aryl group" means an aryl group having 6 or 7 total carbon atoms. Specific examples are: phenyl and 4-methylphenyl groups. As employed in the specification of the present application, the term "C₆ to C₁₄ aryl group" means an aryl group having 6 to 14 total carbon atoms, examples of which are, in addition to the examples given for the "C₆ or C₇ aryl group" above: 1-naphthyl, 2-naphthyl, biphenyl, 1-anthracenyl, and 2-anthracenyl groups.

As employed in the specification of the present application, the term "C₇ or C₈" aralkyl group" means an aralkyl group having 7 or 8 total carbon atoms, specific examples of which are benzyl and 4-methylbenzyl groups. As employed in the specification of the present application, the term "C₇ to C₂₂ aralkyl group" means an aralkyl group having a total of 7 to 22 total carbon atoms. Examples are, in addition to those given for the "C₇ or C₈ aralkyl group" above: naphthylmethyl and trityl groups.

As employed in the specification of the present application, the term "C₈ or C₉ aralkyloxyalkyl group" means an aralkyloxyalkyl group having 8 or 9 total carbon atoms. Specific examples are: benzyloxymethyl, 4-methylphenylmethyloxymethyl, and phenylethyloxymethyl groups. As employed in the specification of the present application, the term "C₈ to C₁₅ aralkyloxyalkyl group" means an aralkyloxyalkyl group having a total of 8 to 15 total carbon atoms. Examples are, in addition to those given for the "C₈ or C₉" aralkyloxyalkyl group" above: 1-naphthylmethyloxymethyl and 2-naphthylmethyloxymethyl groups.

As employed in the specification of the present application, the term "C₁ to C₃ alkanol " means a compound in which a hydroxyl group is bonded to the terminal of a "C₁ to C₃ alkyl group." Specific examples are methanol, ethanol, n-propanol, and isopropanol.

Specific examples of the term " perhalogenate salts " as employed in the specification of the present application are: potassium periodate, sodium periodate, potassium perchlorate, and sodium perchlorate.

As employed in the specification of the present application, the term "hydrogen source" means a compound comprising a hydride or a compound yielding a hydride in a decomposition reaction. Specific examples are: formic acid, trimethylsilane, triethylsilane, tributyltin hydride, sodium borohydride, and lithium aluminium hydride.

As employed in the specification of the present application, the term "noble metal catalyst" means a noble metal itself, such as palladium, platinum or rhodium; a salt thereof; and as necessary, a noble metal or a salt thereof, forming a complex with a ligand (such as acetylacetone and triphenylphosphine). Specific examples are: palladium, platinum, rhodium, palladium acetate, palladium chloride, and tetrakis(triphenylphosphine)palladium.

As employed in the specification of the present application, the term "hydroboronating agent" means a boron compound having 1 to 3 hydrides on a boron atom. Specific examples are: borane, thexylborane, disiamyl borane, catechol borane, 9-borabicyclo[3.3.1]nonane, (+)-isopinocanphenylborane, and (-)-isopinocanphenylborane.

As employed in the specification of the present application, the term "optionally substituted C₁ to C₁₁ organic acid" means a carboxylic acid or sulfonic acid having 1 to 11 total carbon atoms. Specific examples are: formic acid, acetic acid, propionic acid, pivalic acid, cyclopropanecarboxylic acid, cyclobutanecarboxylic acid, cyclopentanecarboxylic acid, cyclohexanecarboxylic acid, benzoic acid, mesitylacetic acid, mesitylsulfonic acid, naphthoic acid, methanesulfonic acid, and p-toluenesulfonic acid.

As employed in the specification of the present application, the substituent referred to in the term "optionally substituted (may have a substituent(s))" means, for example, one or more selected from among:
(1) a halogen atom;
(2) a hydroxyl group;
(3) a thiol group;
(4) a nitro group;
(5) a nitroso group;
(6) a cyano group;
(7) a carboxyl group;
(8) a hydroxysulfonyl group;
(9) an amino group;
(10) a C₁ to C₈ alkyl group (such as a methyl, ethyl, n-propyl, isopropyl, n-butyl, isobutyl, sec-butyl or tert-butyl group);

(11) an unsaturated C₂ to C₈ alkyl group (such as a vinyl, allyl, 1-propenyl, isopropenyl, ethynyl, 1-propynyl, 2-propynyl, 1-butynyl, 2-butynyl or 3-butynyl group);
(12) a C₆ to C₁₄ aryl group (such as a phenyl, 1-naphthyl or 2-naphthyl group); (13) a heteroaryl group with a 5 to 14-membered ring (such as a thienyl, furyl, pyridyl, pyridazyl, pyrimidyl or pyrazyl group);
(14) a nonaromatic heterocyclic group with a 3 to 14-membered ring (such as an azyridinyl, azetidyl, pyrrolidinyl, pyrrolyl, piperidinyl, piperazinyl, homopiperidyl, homopiperazyl, imidazolyl, pyrazolidyl, imidazolidyl, morpholyl, thiomorpholyl, imidazolinyl, oxazolinyl or quinuclidyl group);

(15) a C₃ to C₁₄ cycloalkyl group (such as a cyclopropyl, cyclobutyl, cyclopentyl, cyclohexyl, cycloheptyl or cyclooctyl group);
(16) a C₁ to C₈ alkoxy group (such as a methoxy, ethoxy, n-propoxy, isopropoxy, sec-propoxy, n-butoxy, isobutoxy or tert-butoxy group);
(17) an unsaturated C₂ to C₈ alkoxy group (such as a vinyloxy, allyloxy, 1-propenyloxy, 2-propenyloxy, isopropenyloxy, ethynyloxy, 1-propynyloxy, 2-propynyloxy, 1-butynyloxy or 2-butynyloxy group);
(18) a C₆ to C₁₄ aryloxy group (such as a phenyloxy, 1-naphthyloxy or 2-naphthyloxy group);

(19) a C₇ to C₁₄ aralkyloxy group (such as a benzyloxy, phenethyloxy, 3-phenylpropyloxy, 4-phenylbutyloxy, 1-naphthylmethyloxy or 2-naphthylmethyloxy group);
(20) a heteroaralkyloxy group with a 5 to 14-membered ring (such as a thienylmethyloxy, furylmethyloxy, pyridylmethyloxy, pyridazylmethyloxy, pyrimidylmethyloxy or pyrazylmethyloxy group);
(21) a heteroarylloxy group with a 5 to 14-membered ring (such as a thienyloxyl, furyloxy, pyridyloxy, pyridazyloxy, pyrimidyloxy or pyrazyloxy group);
(22) an aliphatic C₁ to C₉ acyl group (such as an acetyl, propionyl, butyryl, isobutyryl, valeryl, iso-valeryl or pivalyl group);

(23) an aromatic C₇ to C₁₅ acyl group (such as a benzoyl, 1-naphthoyl or 2-naphthoyl group);
(24) an aliphatic C₂ to C₉ acyloxy group (such as an acetoxy, propionyloxy or butyryloxy group);
(25) a C₂ to C₉ alkoxycarbonyl group (such as a methoxycarbonyl, ethoxycarbonyl, n-propoxycarbonyl, isopropoxycarbonyl, n-butoxycarbonyl, isobutoxycarbonyl, sec-butoxycarbonyl or tert-butoxycarbonyl group);
(26) an unsaturated C₃ to C₉ alkoxycarbonyl group (such as a vinyloxycarbonyl, allyloxycarbonyl, 1-propenyloxycarbonyl, isopropenyloxycarbonyl, propargyloxycarbonyl or 2-butynyloxycarbonyl group);

(27) a C₁ to C₈ alkylthio group (such as a methylthio, ethylthio, n-propylthio or iso-propylthio group);
(28) a C₁ to C₈ alkylsulfinyl group (such as a methylsulfinyl, ethylsulfinyl, n-propylsulfinyl or iso-propylsulfinyl group);
(29) a C₁ to C₈ alkylsulfonyl group (such as a methylsulfonyl, ethylsulfonyl, n-propylsulfonyl or iso-propylsulfonyl group);
(30) a C₆ to C₁₄ arylsulfonyl group (such as a benzenesulfonyl, toluenesulfonyl, 1-naphthalenesulfonyl or 2-naphthalenesulfonyl group);

(31) a C₁ to C₈ alkylsulfonyloxy group (such as a methylsulfonyloxy, ethylsulfonyloxy, n-propylsulfonyloxy or isopropylsulfonyloxy group); or
(32) a carbamoyl group.
   Desirable examples are amino groups, C₁ to C₈ alkyl groups, unsaturated C₂ to C₈ alkyl groups, C₆ to C₁₄ aryl groups, heteroaryl groups with 5 to 14 membered rings, nonaromatic heterocyclic groups with 3 to 14-membered rings, and C₃ to C₁₄ cycloalkyl groups. Of these, preferred examples are amino groups, C₁ to C₈ alkyl groups, nonaromatic heterocyclic groups with 3 to 14-membered rings, and C₃ to C₁₄ cycloalkyl groups. The amino group of (9) and the carbamoyl group of (32) given as examples of substituents relating to the term "optionally substituted" may be further substituted with one or two C₁ to C₈ alkyl groups, unsaturated C₂ to C₈ alkyl groups, or C₆ to C₁₄ aryl groups.

The process for producing according to the present invention via a producing intermediate in the form of the 1-hydroxy-19-norvitamin D derivative denoted by formula (X) permits the efficient production of 1-hydroxy-19-nor type 25-hydroxyvitamin D derivative from a starting material in the form of 25-hydroxyvitamin D.

### [Best Modes of Carrying Out the Invention]

Typical examples of the producing process of the present invention will be described below with reference to the following synthesis scheme:

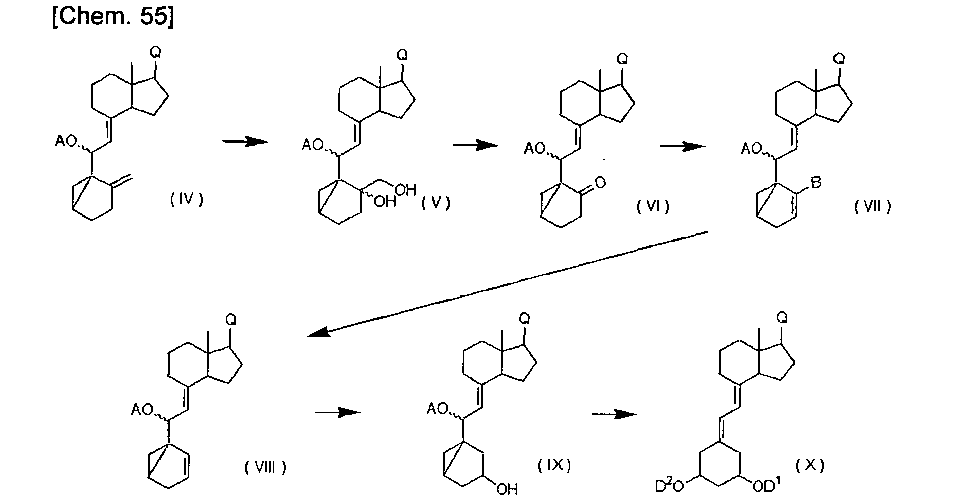

### a) Method of producing the 10,19-diolcyclovitamin D derivative of formula (V) (Step 1)

The 10,19-diolcyclovitamin D derivative denoted by formula (V) can be obtained by subjecting the cyclovitamin D derivative of formula (IV) to an oxidation reaction with an oxidizing agent in a suitable solvent. Examples of oxidizing agents that are suitable for use are: osmium tetraoxide, permanganates (such as potassium permanganate and sodium permanganate), and permanganates (such as potassium permanganate and sodium permanganate) supported on silica gel, cerite, alumina, montmorillonite or the like. It suffices to gradually add an approximately 0.8 to 10 equivalent to the compound of formula (IV) and stir the mixture. The solvent is not specifically limited other than that it not negatively affect the reaction. For example, basic organic solvents such as pyridine and alcohols such as methanol and ethanol are desirably employed. The reaction period is desirably 0.5 to 24 hours, and the reaction temperature is desirably -20 to 80°C.

### b) Method of producing the 10-oxocyclovitamin D derivative of formula (VI) (Step 2)

The 10-oxocyclovitamin D derivative denoted by formula (VI) can be obtained by subjecting the 10,19-diolcyclovitamin D derivative denoted by formula (V) to a carbon-carbon bond cleavage reaction with an oxidizing agent in a suitable solvent. For example, oxidizing agents such as lead tetraacetate, perhalogenate salts (such as potassium periodate and sodium periodate), perhalogenate salts (such as potassium periodate and sodium periodate) supported on silica gel or the like, and metaperiodate salts are desirably employed. It suffices to add the oxidizing agent in an approximately 1 to 10 equivalent to the cyclovitamin D derivative of formula (V) above and stirred the mixture. A solvent in the form of an alcohol solvent such as methanol or ethanol, purified water, or a mixture thereof can be employed. The reaction period is desirably 1 to 24 hours, and the reaction temperature is desirably -20 to 80°C.

### c) Method of producing the cyclovitamin D derivative of formula (VII) (Step 3)

The cyclovitamin D compound of formula (VII) can be obtained by reacting the 10-oxocyclovitamin D derivative of formula (VI) with a halogenating agent, or a base and a sulfonylating agent, in a suitable solvent. A base in the form of lithium hexamethyldisilazide, lithium diisopropylamide, lithium dicyclohexylamide, or the like; a halogenating agent in the form of phosphorus oxychloride, phosphorus pentachloride, or the like; or a sulfonylating agent in the form of trifluoromethanesulfonic anhydride, trifluoromethanesulfonylchloride, N-phenylbis(trifluoromethanesulfonimide), 2[N,N-bis(trifluoromethylsulfonyl)amino]pyridine, 2-[N,N-bis((trifluoromethylsulfonyl)amino]-5-chloropyridine or the like can be employed. It suffices to add the halogenating agent in an approximately 1 to 2 equivalent, the base in a 1 to 5 equivalent, and the sulfonylating agent in a 1 to 5 equivalent to the compound of formula (VI) and conduct a reaction. The solvent is not specifically limited other than that it not negatively affect the reaction. For example, halomethane-based organic solvents such as dichloromethane; ether-based solvents such as dimethyl ether, diethyl ether, diisopropyl ether, t-butylmethyl ether, dimethoxyethane, tetrahydrofuran and 1,4-dioxane; hydrocarbon-based solvents such as toluene, hexane and heptane; and the like can be employed. The reaction period is desirably 1 to 24 hours and the reaction temperature is desirably -80°C to room temperature.

### d) Method of producing the 1,10-olefincyclovitamin D derivative of formula (VIII) (Step 4)

The 1,10-olefincyclovitamin D derivative denoted by formula (VIII) can be obtained by reacting the cyclovitamin D derivative of formula (VII) with an amine and a hydrogen source in the presence of a noble metal catalyst in a suitable solvent. For example, an amine in the form of triethylamine, tripropylamine, tributylamine, or the like; a hydrogen source in the form of formic acid, trimethylsilane, triethylsilane, tributyltin hydride, sodium borohydride, lithium aluminium hydride, or the like; and a noble metal catalyst in the form of palladium, platinum, rhodium, palladium acetate, palladium chloride, tetrakis(triphenylphosphine)palladium, or the like can be employed. It suffices to add the amine in an approximately 1 to 5 equivalent, the hydrogen source in a 1 to 5 equivalent, and the noble metal catalyst in a 0.1 to 5 equivalent to the compound of formula (VII) and conduct a reaction. The solvent is not specifically limited other than that it not negatively affect the reaction. For example, amide-based solvents such as N,N-dimethylformamide and N,N-dimethylacetamide, and ether-based solvents such as tetrahydrofuran and 1,4-dioxane can be employed. The reaction period is desirably 1 to 5 hours and the reaction temperature is desirably 40 to 80°C.

### e) Method of producing 1-hydroxycyclovitamin D derivative of formula (IX) (Step 5)

The 1-hydroxycyclovitamin D derivative denoted by formula (IX) can be obtained by reacting the 1,10-olefincyclovitamin D derivative of formula (VIII) with a hydroboronating agent in a suitable solvent, and then oxidizing the product. A hydroboronating agent in the form of borane, thexylborane, disiamylborane, catechol borane, 9-borabicyclo[3.3.1]nonane, (+)-isopinocanphenylborane, (-)-isopinocanphenylborane, (+)-diisopinocanphenylborane, (-)-diisopinocanphenylborane, or the like can be employed. The hydroboronating agent can be employed in a 1 to 6 equivalent relative to the compound of formula (VIII). The solvent is not specifically limited other than it not negatively affect the reaction; ether-based solvents such as dimethyl ether, diethyl ether, isopropyl ether, tert-butylmethyl ether, dimethoxyethane, tetrahydrofuran and 1,4-dioxane; and hydrocarbon-based solvents such as toluene, hexane and pentane can be employed. The hydroboronation reaction period is desirably 1 to 24 hours, and the reaction temperature desirably falls within a range of -78°C to room temperature. In the oxidation reaction, a mixture of about 30 percent hydrogen peroxide aqueous solution and 1 to 6 mol/L of sodium hydroxide aqueous solution or potassium hydroxide aqueous solution is employed in a proportion of 5 to 20-fold the quantity of the compound of formula (VIII). The reaction period is desirably 0.5 to 6 hours, and the reaction temperature is desirably -78 to 40°C.

### f) Method of producing the 1-hydroxy-19-norvitamin D of formula (X) (Step 6)

The 1-hydroxy-19-norvitamin D derivative denoted by formula (X) can be obtained by opening the cyclopropane ring of the 1-hydroxycyclovitamin D derivative of formula (IX) with an optionally substituted C₁ to C₁₁ organic acid in a suitable solvent. Examples of the optionally substituted C₁ to C₁₁ organic acid are: formic acid, acetic acid, propionic acid, and pivalic acid. The solvent is not specifically limited other than it not negatively affect the reaction. Reactive agents such as formic acid, acetic acid, propionic acid, and pivalic acid that are themselves C₁ to C₁₁ organic acids are desirably employed. The reaction period is desirably 0.1 to 6 hours. The reaction temperature is desirably room temperature to about 80°C.

Following completion of the reaction, the target compounds of the various above reactions can be collected by the usual processes from the reaction mixture. For example, when insoluble matter is present, it can be removed by suitable filtration, and the solvent distilled off under reduced pressure. Alternatively, the reaction mixture can be diluted with an organic solvent such as ethyl acetate, the diluted mixture washed with water, the organic layer dried over anhydrous magnesium sulfate or the like, and the solvent distilled off, yielding the target compound. As needed, purification can be conducted by the usual processes, such as column chromatography, thin layer chromatography, high-performance liquid chromatography, or crystallization.

Among cyclovitamin D derivatives denoted by formula (IV), 25-hydroxy-form cyclovitamin D derivatives can be manufactured by a process following the known scheme shown below by converting the hydroxyl group at position 3 in the 25-hydroxyvitamin D denoted by formula (I) to a sulfonic acid ester such as a tosyl ester and, as needed, introducing a protective group onto the hydroxyl group at position 25, and then adding a C₁ to C₃ alkanol under basic conditions.

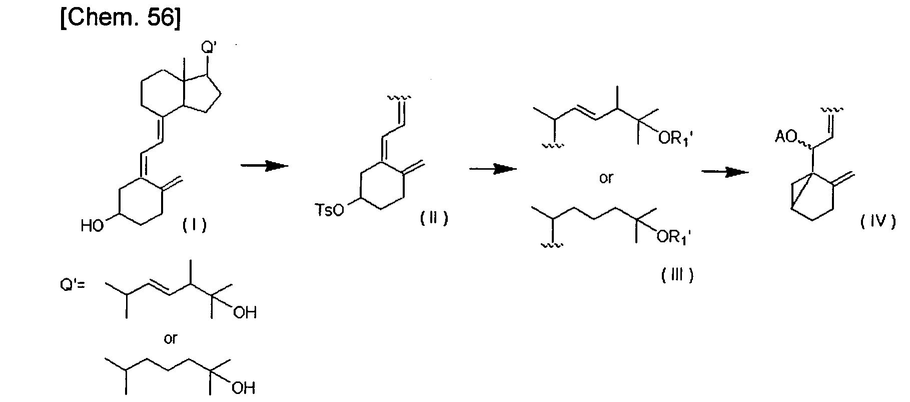

In this scheme, Ts denotes a tosyl group and R^{1'} and A are as defined above.

1-hydroxy-19-nor type 25-hydroxyvitamin D derivative can be prepared by known process that cleavage of protective groups at1,3 and 25 positions, from 1-hydroxy-19-norvitamin D derivative of formula (X).

The conversion rate of a 25-hydroxyvitamin D compound to a 1-hydroxy-19-nor type 25-hydroxyvitamin D compound by the producing process of the present invention is about 1.5 to 2-fold that of conventional processes (see Patent Document 1 and Nonpatent Documents 1 and 2), which is extremely good. In particular, the reaction of Step 5 of the present invention affords good stereoselectivity for position 1, preferentially yielding the targeted isomer. Selecting a highly bulky hydroboronating agent as the reducing agent preferentially yields 1-a(*alpha*)-isomer. Conversely, selecting a hydroboronating agent that is not highly bulky increases the proportion of 1-β(*beta*)-isomer. Suitably selecting the hydroboronating agent in this reaction makes it possible to efficiently obtain a targeted isomer. In the conventional process, in the course of introducing a hydroxyl group at the 1a(*alpha*) position by oxidation; 1β(beta) hydroxyrated compounds and ketones from futrher oxidation were obtained as by-products. (1β(*beta*) hydroxyrated compounds < 5 percent, ketones 20 percent and 50 percent of the targeted compound). The loss in this step is thus great. Further, the reaction progresses via reaction intermediates in the form of ketones, presenting the possibility of losing the asymmetry that has been so painstakingly introduced. However, in the producing process of the present invention, the reaction does not pass through intermediates with such undesirable properties, which is highly advantageous relative to the conventional process.

### [Embodiments]

The present invention is described below through embodiments. However, the present invention is not limited to these Embodiments.

### Example 1 (Embodiment): Synthesis of compound (5a)

Pyridine (5.0 mL) was added to crude compound 4a (0.80 g, 1.8 mmol) at 0°C, a pyridine (5.0 mL) solution of osmium tetraoxide (0.41 g, 1.6 mmol) was added dropwise, and the mixture was stirred at room temperature for 30 minutes. The solution was adjusted to 0°C, 100 mL of 10 percent sodium hydrogensulfite aqueous solution was added, and the mixture was stirred at room temperature for 40 minutes, resulting in termination of the reaction. The reaction solution was extracted with ethyl acetate (50 mL x 3 times), and the organic layer was successively washed with water (20 mL x 2 times), 2 mol/L hydrochloric acid (20 mL x 2 times), saturated sodium hydrogencarbonate aqueous solution (20 mL x 2 times), and saturated sodium chloride aqueous solution (20 mL x 2 times). The organic layer was dried over anhydrous sodium sulfate, filtered, and concentrated, yielding a crude compound (5a) (0.82 g) in the form of a brown, amorphous product.

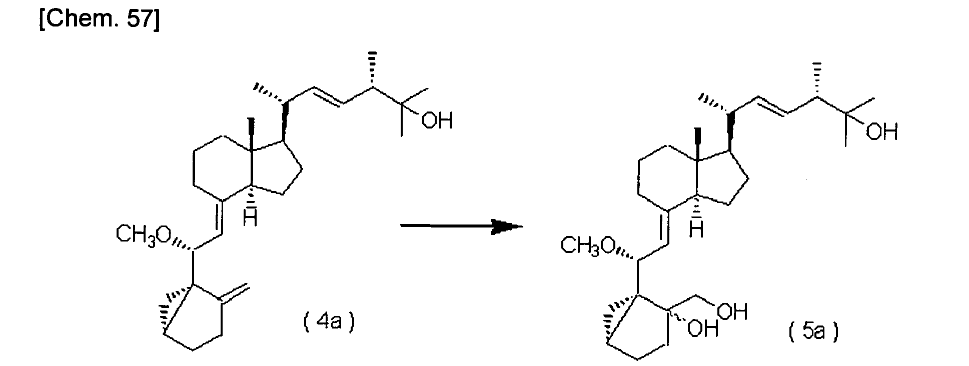

### Example 2 (Embodiment): Synthesis of compound (6a)

Crude compound (5a) (0.82 g) obtained in Example 1 was dissolved in methanol (5.0 mL) at 0°C. An aqueous solution (7 mL) of sodium periodate (1.13 g, 5.2 mmol) was added dropwise and the mixture was stirred at 0°C for 2.5 hours, at which time the reaction ended. The reaction solution was concentrated, diluted with water (150 mL), and extracted with ethyl acetate (50 mL x 3 times). The organic layer was successively washed with water (30 mL x 3 times), 1 mol/L hydrochloric acid (30 mL), saturated sodium hydrogencarbonate aqueous solution (30 mL), and saturated sodium chloride aqueous solution (30 mL). The organic layer was dried over anhydrous sodium sulfate, filtered, and concentrated. The residue was separated and refined by silica gel column chromatography (silica gel 60N (spherical, neutral, 63 to 210 µm, Kanto Chemical Co., Inc.) 13.5 g, n-hexane/ethyl acetate = 4/1 to 3/1), yielding a slightly yellow oil in the form of the compound (6a) denoted below (279.0 mg, total yield from 25-hydroxyvitamin D₂ of four steps combined: 39 percent).

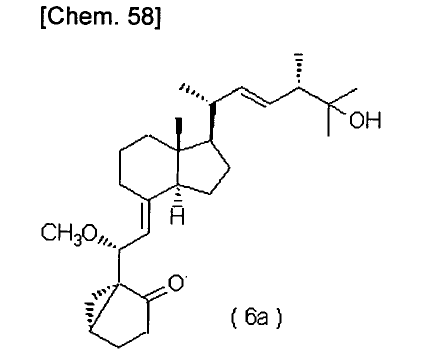

Physical properties of compound (6a)
¹H-NMR (MeOD) δ (ppm) :
5.35(dd,1H,J=8 and 15Hz,H23), 5.27(dd,1H,J=8 and 15Hz,H22),
4.65(d,1H,J=10Hz,H6), 4.37(d,1H,J=10Hz,H7),3.18(s,3H,OMe), 2.67(m,1H,H9a), 2.20(m,2H),2.03(m,7H),1.71(m,3H),1.59-1.17(m,7H),
1.13(s,3H,H26),1.09(s,3H,H27),1.06(t,1H,J=4Hz,H4b),1.04(d,3H,J=7Hz,H21), 0.99(d,3H,J=7Hz,H28),0.57(s,3H,H18)

¹³C-NMR (MeOD) δ (ppm) : 216.73(C10),146.85(C8),138.33(C22),131.55(C23),118.31(C7),74.69(C6), 73.36(C25), 57.79(C17),57.08(C14),56.47(OMe),49.20(C24),46.57(C13), 41.98(C5), 41.84(C20), 41.44(C12),33.93(C1),30.35(C9),28.93(C16),28.32(C26), 26.13(C27),25.77(C3), 24.59(C11),23.15(C15),22.50(C2),21.45(C21),17.98(C4), 15.68(C28),12.74(C18)
ESI-MS : 451.4[M+Na]⁺, 463.4[M+Cl]⁻

### Example 3 (Embodiment): Synthesis of compound (7a)

Lithium hexamethyldisilazide 1.6 mol/L in THF (364 µL, 0.58 mmol) was added to dimethoxyethane (1.0 mL) at -75°C. A methoxyethane (4.0 mL) solution of a portion (100 mg, 0.23 mmol) of the compound (6a) obtained in Example 2 was added dropwise and the mixture was stirred at -75 to 70°C for 1.5 hours. To this solution was added N-phenylbis(trifluoromethanesulfonimide) (205 mg, 0.57 mmol) and the mixture was stirred at -70 to -10 °C for 16 hours, resulting in termination of the reaction. The reaction solution was concentrated, diluted with water (5 mL), and extracted with ethyl acetate (10 mL x 3 times). The organic layer was successively washed with 1 mol/L hydrochloric acid (2 mL), saturated sodium hydrogencarbonate aqueous solution (2 mL), and saturated sodium chloride (2 mL). The organic layer was dried over anhydrous sodium sulfate, filtered, and concentrated, yielding the crude compound (7a) (242.5 mg) denoted below in the form of a brown, amorphous product.

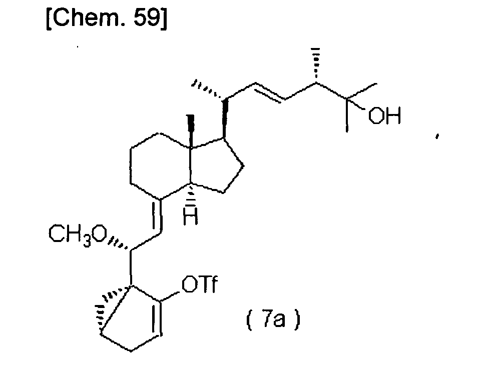

Physical properties of compound (7a)
¹H-NMR (MeOD) δ (ppm) : 5.36(dd,1H,J=8 and 15Hz,H23), 5.33(d,1H,J=2Hz,H1),5.27(dd,1H,J=8 and 15Hz,H22), 4.65(d,1H,J=9Hz,H7),4.37(d,1H,J=9Hz,H6),3.23(s,3H,OMe), 2.59(m,2H), 2.34(dd,1H,J=2 and 18Hz,H2b), 2.05(m,4H),1.75(m,4H),1.48(m,3H), 1.35(m,3H), 1.13(s,3H,H26),1.09(s,3H,H27),1.09(m,1H,H4a), 1.04(d,3H,J=7Hz,H21), 0.99(d,3H,J=7Hz,H28),0.57(s,3H,H18), 0.53(t,1H,J=4Hz,H4b)

¹³C-NMR (MeOD) δ (ppm) : 153.54(C10),146.90(C8),137.93(C22),131.20(C23),117.74(C7),114.61(C1), 76.17(C6), 72.98(C25),57.39(C17),56.70(C14),56.10(OMe),48.62(C24), 46.17(C13), 41.45(C20), 41.02(C12),36.56(C5),30.68(C2),29.97(C9),28.54(C16), 27.94(C26), 25.76(C27), 24.39(C15),22.74(C11),21.07(C21),19.74(C4), 18.21 (C3), 15.31 (C28),12.30(C 18)
ESI-MS : 583.47[M+Na]⁺, 595.59[M+Cl]⁻

### Example 4 (Embodiment): Synthesis of compound (8a)

The crude compound (7a) (242 mg) obtained in Example 3, triphenylphosphine (12.2 mg, 0.046 mmol), and palladium diacetate (5.4 mg, 0.024 mmol) were dissolved in dimethylformamide (1.5 mL). Tributylamine (166 µL, 0.70 mmol) and formic acid (17.6 µm, 0.47 mmol) were successively added dropwise, and the mixture was stirred at 60°C for 50 minutes, resulting in termination of the reaction. The reaction solution was diluted with water (5 mL) and extracted with ethyl acetate (10 mL x 3 times). The organic layer was then successively washed with 1 mol/L hydrochloric acid (2 mL), saturated sodium hydrogencarbonate aqueous solution (2 mL), and saturated sodium chloride aqueous solution (2 mL). The organic layer was dried over anhydrous sodium sulfate, filtered, and concentrated. The residue was separated and purified by preparative TLC (Merck silica gel 60 F254 1.05744, n-hexane/acetone = 3/1), yielding the compound (8a) (20.8 mg, 2 step combined yield of 22 percent from compound (6a) )denoted below in the form of a slightly yellow, amorphous product.

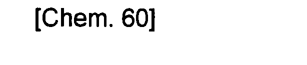

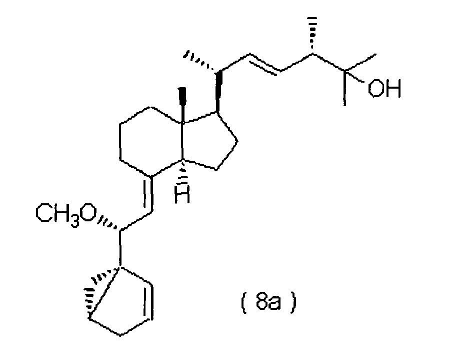

Physical properties of compound (8a)
¹H-NMR (MeOD) δ (ppm) : 5.90(td,1H,J=2 and 5Hz,H10), 5.39(brd,1H,J=5Hz,H1), 5.35(dd,1H,J=8 and 15Hz,H23),5.27(dd,1H,J=8 and 15Hz,H22),4.87(d,1H,J=10Hz,H7), 3.97(d,1H,J=10Hz,H6),3.25(s,3H,OMe), 2.61(dd,1H,J=4 and 11Hz,H9a),2.54(m,1H,H2a), 2.27(brd,1H,J=17Hz,H2b), 2.04(m,4H),1.72(m,4H),1.48(m,3H),1.34(m,3H), 1.13(s,3H,H26), 1.09(s,3H,H27),1.03(m,1H,H4a),1.03(d,3H,J=7Hz,H21), 0.99(d,3H,J=7Hz,H28),0.55(s,3H,H18),0.16(t,1H,J=4Hz,H4b)

¹³C-NMR (MeOD) δ (ppm) : 144.35(C8),138.39(C22),135.41(C10),131.50(C23),128.99(C1),121.07(C7), 80.22(C6), 73.36(C25),57.82(C17),57.10(C14),56.13(OMe),49.22(C24),46.65(C13),41.86(C 20), 41.66(C12),41.32(C5),36.95(C2),30.54(C9),28.99(C16),28.33(C26), 26.12(C27), 25.17(C11),23.08(C15),22.52(C4),21.47(C21),20.01(C3),15.69(C28), 12.52(C18)
ESI-MS : 435.38[M+Na]⁺, 447.43[M+Cl]⁻

### Example 5 (Embodiment): Synthesis of compound (9a)

A portion (13.4 mg, 0.032 mmol) of compound (8a) obtained in Example 4 was dissolved in tetrahydrofuran (THF) (0.5 mL), 9-borabicyclo[3.3.1]nonane 0.5 mol/L in THF (260 µL, 0.13 mmol) was added dropwise at 0°C, and the mixture was stirred at room temperature for 4 hours, resulting in disappearance of the starting material. To the reaction solution were added water (150 µL) and a mixed (1:1) solution (300 µL) of 30 percent hydrogen peroxide aqueous solution and 3 mol/L of sodium hydroxide aqueous solution at 0°C, and the mixture was stirred at room temperature for 1.5 hours. The reaction solution was diluted with water (3 mL) and extracted with ethyl acetate (5 mL x 3 times). The organic layer was then washed with saturated sodium chloride aqueous solution (2 mL). The organic layer was dried over anhydrous sodium sulfate, filtered, and concentrated. The residue was separated and purified by preparative TLC (Merck silica gel 60 F254 1.05744, n-hexane/acetone = 1/1), yielding the compound (9a) (3.2 mg, yield of 23 percent) denoted below in the form of a colorless oil.

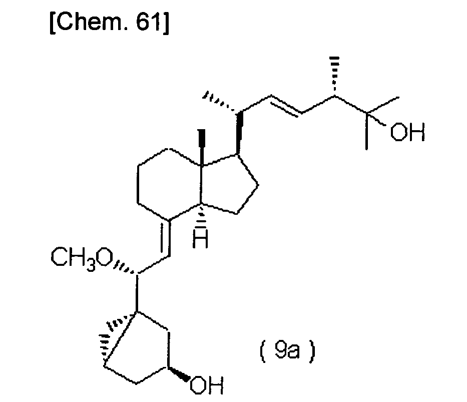

Physical properties of compound (9a)
¹H-NMR (MeOD) δ (ppm) : 5.35(dd,1H,J=8 and 15Hz,H23),5.27(dd,1H,J=8 and 15Hz,H22), 4.94(d,1H,J=9Hz,H7),3.92(m,1H,H1β),3.89(d,1H,J=9Hz,H6), 3.21(s,3H,OMe), 2.64(m,1H,H9a),2.11-1.99(m,6H,H24),1.83-1.26(m,10H), 1.13(m,1H,H3),1.13(s,3H,H26),1.09(s,3H,H27),1.04(d,3H,J=7Hz,H21), 0.99(d,3H,J=7Hz,H28),0.62(s,3H,H18),0.55(dd,1H,J=4 and 8Hz,H4a), 0.37(t,1H,J=4Hz,H4b)

¹³C-NMR (MeOD) δ (ppm) : 144.87(C8),138.38(C22),131.51 (C23),120.41 (C7),81.19(C6),73.35(C25), 71.27(C1), 57.82(C17),57.19(C14),56.20(OMe),49.00(C24),46.59(C13), 41.86(C20), 41.62(C12), 37.91 (C10),37.31 (C2),31.56(C5),30.54(C9),29.00(C16), 28.33(C26),26.12(C27), 25.01(C11),23.15(C15),21.48(C21),19.49(C3), 15.69(C28), 14.80(C4),12.83(C18)
ESI-MS : 453.37[M+Na]⁺, 465.61[M+Cl]⁻

### Example 6 (Embodiment): Synthesis of compounds (10a and 10b)

The compound (9a) (20 mg) obtained in Example 5 was dissolved in acetic acid (0.5 mL) and stirred at 55°C for 20 minutes, resulting in termination of the reaction. The reaction solution was diluted with ice and saturated sodium hydrogencarbonate aqueous solution (5 mL) and extracted with ethyl acetate (5 mL x 3 times). The organic layer was then washed with saturated sodium chloride aqueous solution (2 mL). The organic layer was dried over anhydrous sodium acetate, filtered, and concentrated, yielding a mixture (22 mg) of compounds (10a and 10b).

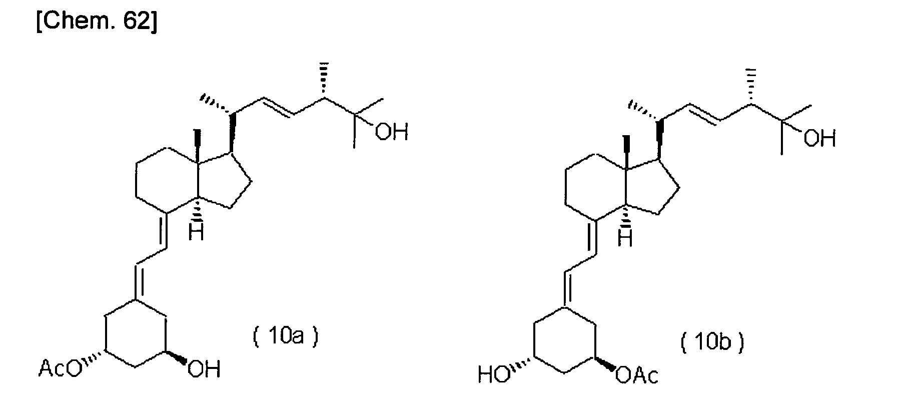

### Example 7 (Reference Example): Synthesis of paricalcitol (12a)

To the mixture (22 mg) of compounds (10a and 10b) obtained in Example 6 was added 10 percent potassium hydroxide ethanol solution (0.5 mL) at 0°C and the mixture was stirred at room temperature for 1 hour, resulting in termination of the reaction. The reaction solution was diluted with water (3 mL) and extracted with ethyl acetate (10 mL x 3 times). The organic layer was washed with saturated sodium chloride aqueous solution (2 mL), dried over anhydrous sodium sulfate, filtered, and concentrated. The residue was separated and purified by preparative TLC (Merck silica gel 60 F254 1.05715, chloroform/methanol = 10/1), yielding paricalcitol (12a) (1.7 mg, three step combined yield from compound 8a of 9 percent) in the form of a white powder.

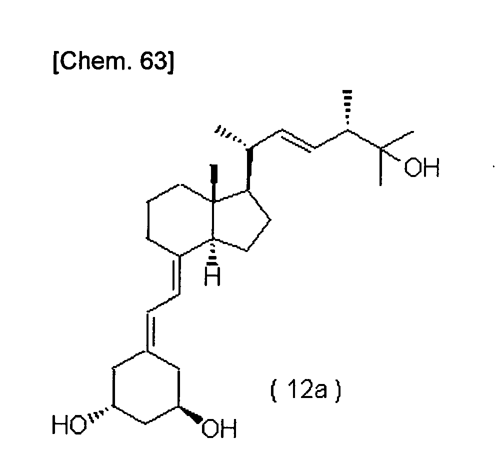

### Physical properties of paricalcitol (12a)

¹H-NMR (MeOD) δ (ppm) : 6.21(d,1H,J=11Hz,H6),5.88(d,1H,J=11Hz,H7),5.35(dd,1H,J=8 and 15Hz,H23), 5.27(dd,1H,J=8 and 15Hz,H22), 4.04(m,1H,H1β),3.98(m,1H,H3a), 2.84(brd,1H,J=11Hz,H9a), 2.59(brd,1H,J=13Hz,H4a), 2.41(brd,1H,J=13Hz,H10a), 2.21(dd,1H,J=8 and 13Hz,H4b),2.16(dd,1H,J=7 and 13Hz,H10b), 2.04(m,4H), 1.84(m,1H,H2a),1.76(m,2H),1.67(m,2H),1.55(m,3H),1.35(m,3H),1.13(s,3H,H26), 1.09(s,3H,H27),1.04(d,3H,J=7Hz,H21),0.99(d,3H,J=7Hz,H28),0.58(s,3H,H18)

¹³C-NMR (MeOD) δ (ppm) 142.07(C8),138.46(C22),133.98(C5),131.45(C23),123.47(C6),117.22(C7), 73.37(C25), 68.04(C1),67.76(C3),57.84(C17),57.63(C14),49.10(C24),46.75(C13), 45.46(C10), 42.74(C2),41.90(C20),41.82(C12),37.69(C4),29.87(C9),29.02(C16), 28.33(C26),26.12(C27),24.55(C11),23.30(C15),21.49(C21),15.70(C28), 12.75(C18)
ESI-MS : 439.32[M+Na]⁺, 452.53[M+Cl]⁻

### Example 8 (Embodiment): Synthesis of compound (5b)

To 1.05 g (2.2 mmol) of the 25-methoxymethyl (MOM) derivative (4b) of compound (4a) was added pyridine (7.0 mL). A pyridine (3.0 mL) solution of osmium tetraoxide (0.53 g, 2.1 mmol) was added dropwise and the mixture was stirred for 2 hours at room temperature. The solution was adjusted to 0°C and 110 mL of 10 percent sodium hydrogensulfite aqueous solution was added. The mixture was stirred at room temperature for 1 hour, resulting in termination of the reaction. The reaction solution was extracted with ethyl acetate (50 mL x 3 times). The organic layer was successively washed with water (30 mL), 1 mol/L hydrochloric acid (20 mL x 3 times), saturated sodium hydrogencarbonate aqueous solution (20 mL), and saturated sodium chloride aqueous solution (20 mL). The organic layer was dried over anhydrous sodium sulfate, filtered, and concentrated, yielding the crude compound (5b) (1.03g) denoted below in the form of a brown amorphous product.

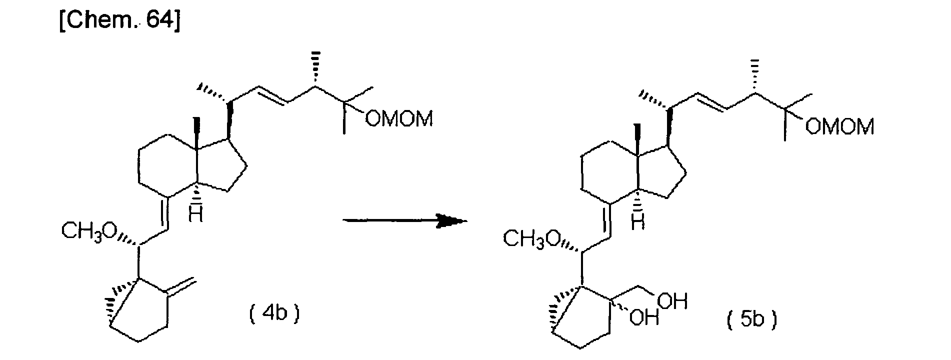

### Physical properties of compound (5b)

¹H-NMR (MeOD) δ (ppm) : 5.36(dd,1H,J=8 and 15Hz,H23), 5.27(dd,1H,J=8 and 15Hz,H22), 4.89(d,1H,J=9Hz,H6),4.70(s,2H,MOM-CH2),4.58(d,1H,J=9Hz,H7), 3.65(d,1H,J=11Hz,H19a),3.58(d,1H,J=11Hz,H19b),3.34(s,3H,MOM-CH3), 3.20(s,3H,OMe),2.71(m,1H,H9a),2.20(m,1H,H24),2.03(m,4H),1.73(m,3H), 1.63(dd,1H,J=7 and 12Hz,H2b),1.56-1.30(m,9H),1.17(s,3H,H26),1.13(s,3H,H27), 1.03(d,3H,J=7Hz,H21),0.99(d,3H,J=7Hz,H28),0.60(s,3H,H18), 0.50(dd,1H,J=5 and 8Hz,H4a), 0.38(t,1H,J=5Hz,H4b)

¹³C-NMR (MeOD) δ (ppm) : 146.78(C8),138.42(C22),131.29(C23),119.22(C7),91.94(MOM-CH2),85.06(C10), 79.45(C25),78.75(C6),68.34(C19),57.77(C17),57.04(C14),55.89(OMe), 55.47(MOM-CH3),48.53(C24),48.11(C13),41.84(C20),41.42(C12),36.49(C5), 33.77(C1), 30.19(C9),28.93(C16),25.27(C2),25.20(C26),24.44(C11),23.61(C27), 23.30(C15), 21.45(C21),21.37(C3),15.67(C28),12.89(C18),11.86(C4)
ESI-MS : 527.56[M+Na]⁺, 539.63[M+Cl]⁻

### Example 9 (Embodiment): Synthesis of compound (6b)

The crude compound (5b) (1.03 g) obtained in Example 8 was dissolved in methanol (10 mL) at 0°C. An aqueous solution (9 mL) of sodium periodate (1.32 g, 6.1 mmol) was added dropwise and the mixture was stirred at 0°C for 2 hours, resulting in termination of the reaction. The reaction solution was concentrated, diluted with water (80 mL) , and extracted with ethyl acetate (50 mL x 3 times). The organic layer was then successively washed with water (30 mL x 3 times), 1 mol/L hydrochloric acid (20 mL), saturated sodium hydrogencarbonate aqueous solution (20 mL), and saturated sodium chloride aqueous solution (20 mL). The organic layer was dried over anhydrous sodium sulfate, filtered, and concentrated. The residue was separated and purified by silica gel column chromatography (silica gel 60N (spherical, neutral, 63-210 µm, Kanto Chemical Co., Inc.) 18.5 g, n-hexane/ethyl acetate = 6/1), yielding the compound (6b) (312.6 mg, combined 5 step yield from 25-hydroxyvitamin D₂ of 29 percent) denoted below in the form of a slightly yellow oil.

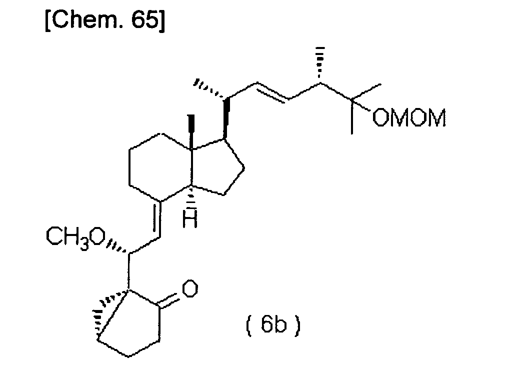

### Physical properties of compound (6b)

¹H-NMR (MeOD) δ (ppm) : 5.35(dd,1H,J=8 and 15Hz,H23),5.27(dd,1H,J=8 and 15Hz,H22),4.70(s,2H,MOM-CH2),4.65(d,1H,J=9Hz,H6), 4.61(d,1H,J=9Hz,H7), 3.33(s,3H,MOM-CH3), 3.18(s,3H,OMe),2.69(m,1H,H9a),2.20(m,3H),2.03(m,6H), 1.74(m,3H),1.56-1.23(m,7H),1.17(s,3H,H26),1.13(s,3H,H27),1.03(d,3H,J=7Hz,H21), 1.03(m,1H,H4b),0.99(d,3H,J=7Hz,H28),0.57(s,3H,H18)

¹³C-NMR (MeOD) δ (ppm) : 216.72(C10),146.83(C8),138.43(C22),131.29(C23),118.32(C7),91.90(MOM-CH2), 79.46(C25),74.69(C6),57.76(C17),57.07(C14),56.47(OMe),55.47(MOM-CH3), 48.11(C24),46.57(C13),41.98(C5),41.84(C20),41.44(C12),33.93(C1),30.35(C9), 28.93(C16),25.78(C3),25.20(C26),24.58(C11),23.61(C27),23.15(C15),22.50(C2), 21.45(C21),17.97(C4),15.67(C28),12.74(C 18)
ESI-MS : 495.33[M+Na]⁺, 507.41[M+Cl]⁻

### Example 10 (Embodiment): Synthesis of compound (7b)

Lithium hexamethyldisilazide 1.6 mol/L in THF (495 µL, 0.79 mmol) was added to dimethoxyethane (1.5 mL) at -75°C, a dimethoxyethane (3.5 mL) solution of compound (6b) (150 mg, 0.31 mmol) was added dropwise, and the mixture was stirred for 1 hour at -75 to -70°C. To this solution was added N-phenylbis(trifluoromethanesulfonimide) (284 mg, 0.79 mmol), and the mixture was stirred at -75 to 15°C for 15.5 hours, resulting in termination of the reaction. The reaction solution was concentrated, diluted with water (5 mL), and extracted with ethyl acetate (10 mL x 3 times). The organic layer was then successively washed with 1 mol/L hydrochloric acid (2 mL), saturated sodium hydrogencarbonate aqueous solution (2 mL), and saturated sodium chloride aqueous solution (2 mL). The organic layer was dried over anhydrous sodium sulfate, filtered, and concentrated, yielding the crude compound (7b) (290.8 mg) denoted below in the form of a brown amorphous product.

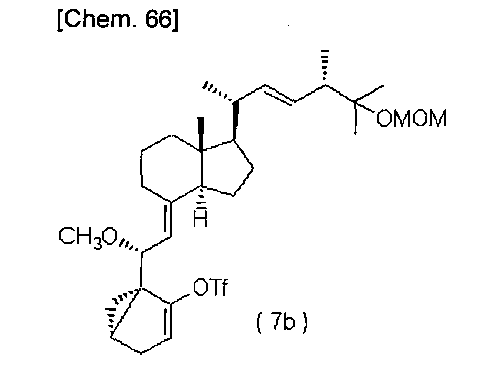

### Physical properties of compound (7b)

¹H-NMR (MeOD) δ (ppm) : 5.36(dd,1H,J=8 and 15Hz,H23), 5.32(d,1H,J=2Hz,H1),5.28(dd,1H,J=8 and 15Hz,H22),4.70(s,2H,MOM-CH2),4.65(d,1H,J=10Hz,H7), 4.37(d,1H,J=10Hz,H6),3.44(s,3H,MOM-CH3),3.23(s,3H,OMe), 2.60(m,2H),2.35(dd,1H,J=2 and 17Hz,H2b), 2.20(m,1H,H24), 2.03(m,3H), 1.75(m,4H),1.45(m,3H),1.35(m,3H),1.28(s,3H,H26),1.17(s,3H,H27), 1.09(dd,1H,J=5 and 8Hz,H4a),1.04(d,3H,J=7Hz,H21),0.99(d,3H,J=7Hz,H28), 0.57(s,3H,H18),0.53(t,1H,J=5Hz,H4b)

¹³C-NMR (MeOD) δ (ppm) : 153.88(C10),147.23(C8),138.37(C22),131.28(C23),118.09(C7),114.97(C1), 91.91(MOM-CH2),79.43(C25),76.51(C6),57.73(C17),57.03(C14),56.44(OMe), 55.44(MOM-CH3),48.08(C24),46.52(C13),41.80(C20),41.36(C12),36.90(C5), 31.02(C2), 30.31(C9),28.88(C16),25.16(C26),24.73(C11),23.58(C27),23.08(C15), 21.41 (C21), 20.08(C4),18.55(C3),15.63(C28),12.65(C18)
ESI-MS : 627.35[M+Na]⁺

### Example 11 (Embodiment): Synthesis of compound (8b)

The crude compound (7b) (290 mg) obtained in Example 10, triphenylphosphine (16.8 mg, 0.063 mmol), and palladium diacetate (7.1 mg, 0.031 mmol) were dissolved in dimethylformamide (4 mL). Tributylamine (227 µL, 0.95 mmol) and formic acid (24 µL, 0.63 mmol) were successively added dropwise at room temperature, and the mixture was stirred at 60°C for 45 minutes, resulting in termination of the reaction. The reaction solution was diluted with water (10 mL) and extracted with ethyl acetate (15 mL x 3 times). The organic layer was successively washed with 1 mol/L hydrochloric acid (3 mL), saturated sodium hydrogencarbonate aqueous solution (3 mL), and saturated sodium chloride aqueous solution (3 mL). The organic layer was then dried over anhydrous sodium sulfate, filtered, and concentrated. The residue was separated and purified by silica gel column chromatography (silica gel 60N (spherical, neutral, 63-210 µm, Kanto Chemical Co., Inc.) 4.0 g, n-hexane/acetone = 50/1), yielding the compound (8b) (71.6 mg, a combined two step yield from compound 6b of 49 percent) in the form of a slightly yellow oil.

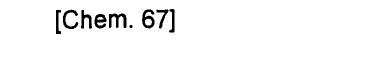

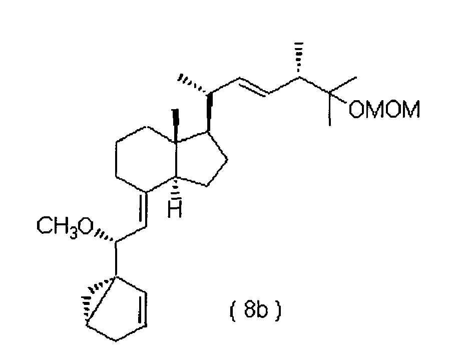

### Physical properties of compound (8b)

¹H-NMR (MeOD) δ (ppm) : 5.90(td,1H,J=2 and 5Hz,H10), 5.39(brd,1H,J=4Hz,H1),5.35(dd,1H,J=8 and 15Hz,H23),5.27(dd,1H,J=8 and 15Hz,H22), 4.87(d,1H,J=10Hz,H7), 4.70(s,2H,MOM-CH2),3.97(d,1H,J=10Hz,H6), 3.33(s,3H,MOM-CH3),3.25(s,3H,OMe),2.61(dd,1H,J=5 and 13Hz,H9a), 2.54(m,1H,H2a), 2.27(brd,1H,J=18Hz,H2b),2.20(m,1H,H24),2.03(m,3H), 1.72(m,2H),1.47(m,4H), 1.34(m,4H),1.17(s,3H,H26),1.13(s,3H,H27), 1.03(m,1H,H4a), 1.03(d,3H,J=7Hz,H21), 0.98(d,3H,J=7Hz,H28),0.55(s,3H,H18), 0.16(t,1H,J=4Hz,H4b)

¹³C-NMR (MeOD) δ (ppm) : 144.29(C8),138.44(C22),135.37(C10),131.20(C23),128.95(C1),121.04(C7), 91.90(MOM-CH2),80.18(C6),79.43(C25),57.77(C17),57.05(C14),56.09(OMe), 55.43(MOM-CH3),48.08(C24),46.61 (C13),41.83(C20),41.61 (C12),41.28(C5), 36.91(C2), 30.50(C9),28.95(C16),25.16(C26),25.13(C11),23.56(C27),23.04(C15), 22.48(C4), 21.42(C21),19.98(C3),15.63(C28),12.48(C18)
ESI-MS : 479.38[M+Na]⁺

### Example 12 (Embodiment): Synthesis of compound (9b)

The compound (8b) (45 mg, 0.098 mmol) obtained in Example 11 was dissolved in THF (1 mL), 9-borabicyclo[3.3.1]nonane 0.5 mol/L in THF (490 µL, 0.25 mmol) was added dropwise at 0°C, and the mixture was stirred for 1.5 hours at room temperature, at which point the starting material had disappeared. Water (300 µL) and a mixed (1:1) solution (600 µL) of 30 percent hydrogen peroxide aqueous solution and 3 mol/L sodium hydroxide aqueous solution were added at 0°C, and the mixture was stirred at room temperature for 30 minutes. The reaction solution was diluted with water (5 mL) and extracted with ethyl acetate (5 mL x 3 times). The organic layer was successively washed with 1 mol/L hydrochloric acid (2 mL), saturated sodium hydrogencarbonate aqueous solution (2 mL), and saturated sodium chloride aqueous solution (2 mL). The organic layer was dried over anhydrous sodium sulfate, filtered, and concentrated. The residue was separated and purified by preparative TLC (Merck silica gel 60 F254 1.05744, n-hexane/acetone = 3/1), yielding the compound (9b) (18.0 mg, yield 39 percent) denoted below in the form of a colorless oil.

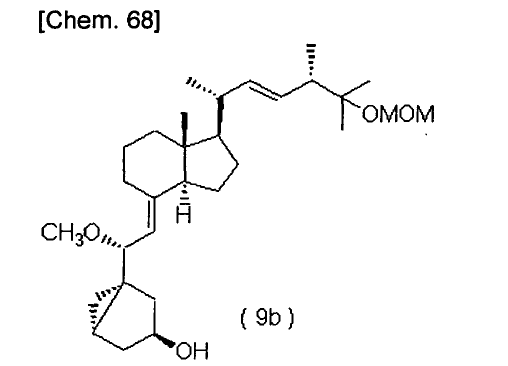

### Physical properties of compound (9b)

¹H-NMR (MeOD) δ (ppm) : 5.36(dd,1H,J=8 and 15Hz,H23), 5.28(dd,1H,J=8 and 15Hz,H22), 4.94(d,1H,J=9Hz,H7),4.70(s,2H,MOM-CH2),3.92(m,1H,H1β), 3.89(d,1H,J=9Hz,H6),3.34(s,3H,MOM-CH3),3.21(s,3H,OMe),2.64(m,1H,H9a), 2.20(m,1H,H24),2.03(m,4H),1.78-1.28(m,12H),1.17(m,1H,H3),1.17(s,3H,H26), 1.13(s,3H,H27),1.04(d,3H,J=7Hz,H21),0.99(d,3H,J=7Hz,H28),0.62(s,3H,H18), 0.55(dd,1H,J=4 and 8Hz,H4a),0.33(t,1H,J=4Hz,H4b)

¹³C-NMR (MeOD) δ (ppm) : 144.86(C8),138.47(C22),131.24(C23),120.41 (C7),91.94(MOM-CH2), 81.19(C6),79.45(C25),71.26(C1),57.76(C17),57.18(C14),56.20(OMe), 55.46(MOM-CH3),48.11(C24),46.58(C13),41.87(C20),41.61(C12),37.90(C10), 37.31(C2),31.56(C5),30.53(C9),29.01(C16),25.20(C26),25.01(C11),23.60(C27), 23.15(C15),21.48(C21),19.49(C3),15.67(C28),14.80(C4),12.83(C18)
ESI-MS : 497.34[M+Na]⁺, 509.35[M+Cl]⁻

### Example 13 (Embodiment): Synthesis of compounds (10c and 10d)

The compound (9b) (17 mg, 0.036 mmol) obtained in Example 12 was dissolved in acetic acid (0.4 mL) and stirred at 60°C for 20 minutes, resulting in termination of the reaction. The reaction solution was diluted with ice and saturated sodium hydrogencarbonate (10 mL), and extracted with ethyl acetate (5 mL x 3 times). The organic layer was successively washed with water (2 mL) and saturated sodium chloride aqueous solution (2 mL). The organic layer was dried over sodium sulfate, filtered and concentrated, yielding a mixture (17.3 mg) of compounds (10c and 10d).

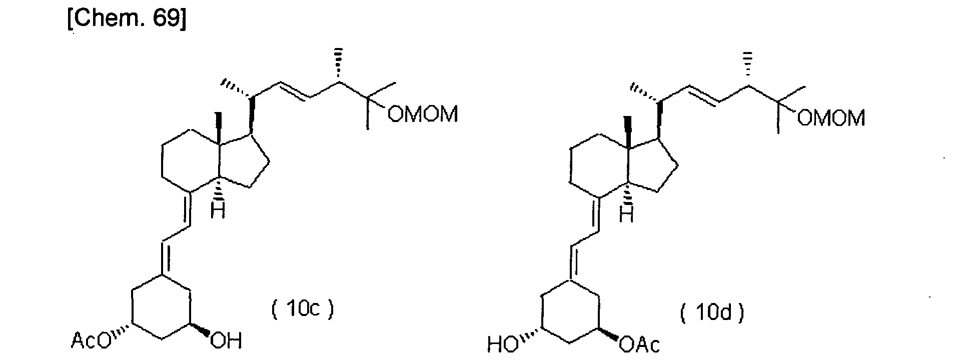

### Example 14 (Reference Example): Synthesis of compound (11 a)

To the mixture (17.3 mg) of compounds (10c and 10d) obtained in Example 13 was added a 10 percent potassium hydroxide ethanol solution (0.5 mL) at 0°C, and the resulting mixture was stirred for 1 hour, resulting in termination of the reaction. The reaction solution was concentrated, diluted with water (5 mL), and extracted with ethyl acetate (5 mL x 3 times). The organic layer was then washed with saturated sodium chloride aqueous solution (2 mL), dried over anhydrous sodium sulfate, filtered, and concentrated, yielding the crude compound (11a) (15.2 mg) denoted below in the form of a colorless amorphous product.

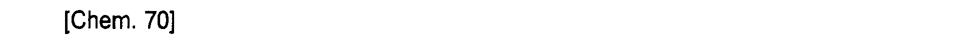

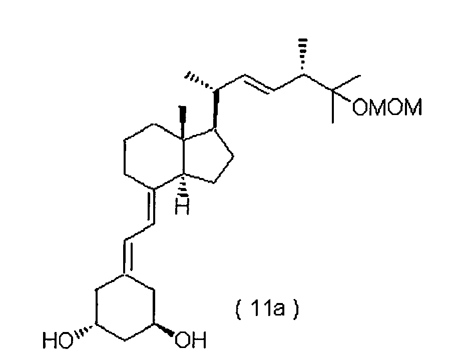

### Physical properties of compound (11 a)

¹H-NMR (MeOD) δ (ppm) : 6.21(d,1H,J=11Hz,H6),5.88(d,1H,J=11Hz,H7),5.35(dd,1H,J=8 and 15Hz,H23),5.28(dd,1H,J=8 and 15Hz,H22),4.70(s,2H,MOM-CH2), 4.03(m,1H,H1β), 3.97(m,1H,H3a),3.34(s,3H,MOM-CH3), 2.83(m,1H,H9a),2.59(dd,1H,J=4 and 13Hz,H4a),2.40(dd,1H,J=4 and 13Hz,H10a),2.18(m,3H),2.02(m,3H), 1.84(m,1H,H2a),1.76(m,2H),1.67(m,2H), 1.52(m,3H),1.30(m,3H),1.17(s,3H,H26), 1.13(s,3H,H27),1.03(d,3H,J=7Hz,H21), 0.99(d,3H,J=7Hz,H28),0.58(s,3H,H18)

¹³C-NMR (MeOD) δ (ppm) : 142.01(C8),138.51(C22),133.95(C5),131.15(C23),123.43(C6),117.19(C7), 91.91 (MOM-CH2),79.44(C25),68.00(C1),67.72(C3),57.79(C17),57.58(C14), 55.43(MOM-CH3), 48.07(C24),46.71 (C13),45.42(C4),42.70(C2),41.87(C20), 41.78(C12), 37.65(C10), 29.82(C9),28.99(C16),25.17(C26),24.51(C11), 23.56(C27), 23.26(C15),21.45(C21), 15.64(C28),12.72(C18)
ESI-MS : 483.41[M+Na]⁺, 495.44[M+Cl]⁻

### Example 15 (Reference Example): Synthesis of paricalcitol (12a)

The crude compound (11 a) (3.9 mg) obtained in Example 14 was dissolved in a mixed solution (1:3) (0.5 mL) of tetrahydrofuran and methanol. Camphor sulfonic acid (5.8 mg, 0.025 mmol) was added at 0°C and the mixture was stirred at room temperature for 3 hours, resulting in termination of the reaction. The reaction solution was diluted with saturated sodium hydrogencarbonate (5 mL) and extracted with ethyl acetate (5 mL x 3 times). The organic layer was then washed with saturated sodium chloride aqueous solution (2 mL). The organic layer was dried over anhydrous sodium sulfate, filtered, and concentrated. The residue was separated and purified by preparative TLC (Merck silica gel 60 F254 1.05715, chloroform/methanol = 10/1), yielding paricalcitol (12a) (1.2 mg, three step combined yield from compound 9b of 31 percent) in the form of a white powder.

### Example 16 (Embodiment): Synthesis of compound (5c)

Ethanol (1.0 mL) was added to crude compound (4c) (21.2 mg, 0.045 mmol) at 0°C. An aqueous solution (0.4 mL) of potassium permanganate (21.5 mg, 0.13 mmol) was added dropwise and the mixture was stirred at room temperature for 4 hours, resulting in termination of the reaction. The reaction solution was filtered, diluted with water (5 mL), and extracted with ethyl acetate (5 mL x 3 times). The organic layer was then washed with saturated sodium chloride aqueous solution (1 mL). The organic layer was dried over anhydrous sodium sulfate, filtered, and concentrated. The residue was separated and purified by preparative TLC (Merck silica gel 60 F254 1.05715, n-hexane/ethyl acetate = 1/1), yielding compound (5c) (3.8 mg, four step combined yield from 25-hydroxyvitamin D₂ of 20 percent) in the form of a white powder.

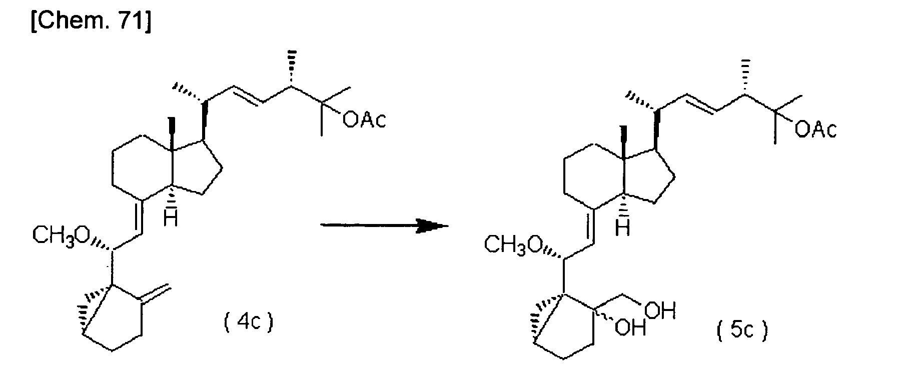

### Physical properties of compound (5c)

¹H-NMR (MeOD) δ (ppm) : 5.31(m,2H,H22 and H23),4.89(d,1H,J=9Hz,H6), 4.58(d,1H,J=9Hz,H7),3.65(d,1H,J=11Hz,H19a), 3.58(d,1H,J=11Hz,H19b), 3.20(s,3H,OMe), 2.72(m,2H,H9a and H24), 2.03(m,4H),1.93(s,3H,Ac), 1.72(m,3H), 1.63(dd,1H,J=7 and 12Hz,H2b), 1.58-1.28(m,9H), 1.39(s,3H,H26),1.38(s,3H,H27),1.04(d,3H,J=7Hz,H21), 0.98(d,3H,J=7Hz,H28), 0.60(s,3H,H18),0.51(dd,1H,J=4 and 8Hz,H4a), 0.38(t,1H,J=4Hz,H4b)

¹³C-NMR (MeOD) δ (ppm) : 172.47(Ac-CO),146.75(C8), 139.37(C22), 130.09(C23),119.24(C7),86.10(C25),85.06(C10), 78.73(C6),68.33(C19), 57.66(C17),57.01(C14),55.89(OMe),46.54(C13),46.45(C24),41.80(C20), 41.40(C12), 36.48(C5),33.76(C1),30.18(C9),28.89(C16),25.27(C2), 24.43(C11),24.33(C26),23.61(C27),23.29(C15),22.42(Ac),21.45(C21),21.33(C3), 15.76(C28),12.89(C18),11.85(C4)
ESI-MS : 525.43[M+Na]⁺, 537.44[M+Cl]⁻

### Example 17 (Embodiment): Synthesis of compound (6c)

The crude compound (6a) (1.26 g) was dissolved in dichloromethane (10.0 mL) at 0°C. Pyridine (10.0 mL, 0.12 mol), acetic anhydride (6.0 mL, 0.058 mol), and 4-dimethylaminopyridine (0.35 g, 2.9 mmol) were added, the mixture was stirred for 30 minutes at room temperature and then at 40°C for 10 minutes, resulting in termination of the reaction. The reaction solution was concentrated, diluted with water (50 mL), and extracted with ethyl acetate (50 mL x 3 times). The organic layer was successively washed with 1 mol/L hydrochloric acid (20 mL x 4 times), saturated sodium hydrogencarbonate aqueous solution (10 mL x 2 times), and saturated sodium chloride aqueous solution (10 mL x 2 times). The organic layer was dried over anhydrous sodium sulfate, filtered, and concentrated. The residue was separated and purified by silica gel column chromatography (silica gel 60N (spherical, neutral, 63-210 µm, Kanto Chemical Co., Inc.) 26.0 g, n-hexane/acetone = 8/1 to 5/1), yielding compound (6c) (276.8 mg, five step combined yield from 25-hydroxyvitamin D₂ of 15 percent) in the form of a slightly yellowish oil.

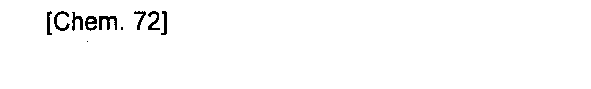

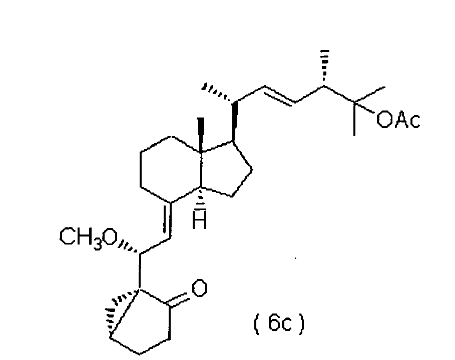

### Physical properties of compound (6c)

¹H-NMR (MeOD) δ (ppm) : 5.32(m,2H,H22 and H23), 4.65(d,1H,J=9Hz,H6),4.61(d,1H,J=9Hz,H7), 3.18(s,3H,OMe),2.70(m,2H,H9a and H24), 2.22(m,2H),2.01(m,6H), 1.93(s,3H,Ac),1.74(m,3H),1.56-1.24(m,7H), 1.39(s,3H,H26),1.38(s,3H,H27), 1.03(d,3H,J=7Hz,H21),1.03(m,1H,H4b), 0.98(d,3H,J=7Hz,H28),0.58(s,3H,H18)

¹³C-NMR (MeOD) δ (ppm) : 215.20(C10),170.95(Ac-CO), 145.27(C8), 137.84(C22),128.56(C23),116.81(C7),84.58(C25),73.15(C6),56.12(C17), 55.52(C14),54.95(OMe),45.04(C13),44.92(C24),40.45(C5),40.26(C20), 39.89(C12), 32.40(C1),28.81(C9),27.36(C16),24.25(C3),23.05(C11),22.80(C26), 22.08(C27), 21.61(C15),20.97(C2),20.89(Ac-Me),19.92(C21), 16.44(C4), 14.23(C28), 11.21(C18)
ESI-MS : 493.41[M+Na]⁺

### Example 18 (Embodiment): Synthesis of compound (7c)

Lithium hexamethyldisilazide 1.6 mol/L in THF (135 µL, 0.21 mmol) was added to dimethoxyethane (1.0 mL) at -75°C. A dimethoxyethane (2.0 mL) solution of compound (6c) (51.0 mg, 0.11 mmol) was added dropwise, and the mixture was stirred for 2 hours at -75°C. To this solution was added N-phenylbis (trifluoromethanesulfonimide) (77 mg, 0.21 mmol), and the mixture was stirred at -75 to 20°C for 20 hours, resulting in termination of the reaction. The reaction solution was concentrated, diluted with water (5 mL), and extracted with ethyl acetate (5 mL x 3 times). The organic layer was successively washed with 1 mol/L hydrochloric acid (2 mL), saturated sodium hydrogencarbonate aqueous solution (2 mL), and saturated sodium chloride aqueous solution (2 mL). The organic layer was dried over anhydrous sodium sulfate, filtered, and concentrated, yielding the crude compound (7c) (87.8 mg) denoted below in the form of a yellow amorphous product.

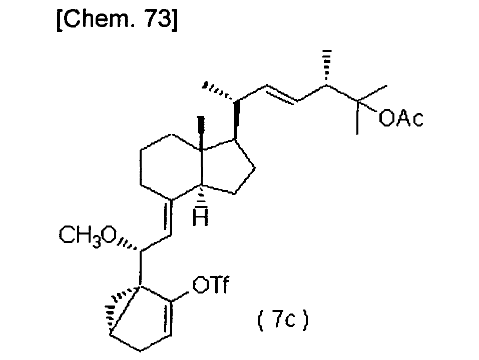

### Physical properties of compound (7c)

¹H-NMR (MeOD) δ (ppm) : 5.32(m,3H,H1,H22 and H23), 4.65(d,1H,J=9Hz,H7),4.37(d,1H,J=9Hz,H6),3.23(s,3H,OMe),2.72(m,1H,H24), 2.59(m,2H),2.34(dd,1H,J=2 and 18Hz,H2b), 2.02(m,3H),1.93(s,3H,Ac), 1.76(m,4H), 1.47(m,3H),1.39(s,3H,H26), 1.38(s,3H,H27),1.34(m,3H), 1.09(dd,1H,J=4 and 8Hz,H4a),1.03(d,3H,J=7Hz,H21), 0.98(d,3H,J=7Hz,H28), 0.57(s,3H,H18),0.53(t,1H,J=4Hz,H4b)

¹³C-NMR (MeOD) δ (ppm) : 172.40 (Ac-CO),153.87(C10),147.20(C8),139.31(C22), 130.08(C23),118.11 (C7), 114.98(C1),86.08(C25),76.52(C6),57.62(C17),57.01(C14), 56.44(OMe), 46.52(C13),46.42(C24),41.75(C20),41.34(C12),36.90(C5),31.02(C2), 30.30(C9),28.85(C16),24.72(C11),24.29(C26),23.57(C27),23.07(C15), 22.39(AcMe), 21.40(C21),20.08(C4),18.56(C3),15.72(C28),12.64(C18)
ESI-MS : 625.35[M+Na]⁺ 637.33[M+Cl]⁻

### Example 19 (Embodiment): Synthesis of compound (8c)

The crude compound (7c) (87 mg) obtained in Example 18, triphenylphosphine (6.8 mg, 0.026 mmol), and palladium diacetate (2.5 mg, 0.011 mmol) were dissolved in dimethylformamide (1 mL). Tributylamine (77 µL, 0.32 mmol) and formic acid (8.2 µL, 0.21 mmol) were successively added dropwise at room temperature, and the mixture was stirred at 60°C for 1 hour, resulting in termination of the reaction. The reaction solution was diluted with water (3 mL) and extracted with ethyl acetate (5 mL x 3 times). The organic layer was then successively washed with 1 mol/L hydrochloric acid (2 mL), saturated sodium hydrogencarbonate aqueous solution (2 mL), and saturated sodium chloride aqueous solution (2 mL). The organic layer was dried over anhydrous sodium sulfate, filtered, and concentrated. The residue was separated and purified by preparative TLC (Merck silica gel 60 F254 1.05744, n-hexane/acetone = 10/1), yielding the compound (8c) (4.6 mg, 2 step combined yield from compound 6c of 9 percent) denoted below in the form of a slightly yellow, amorphous product.

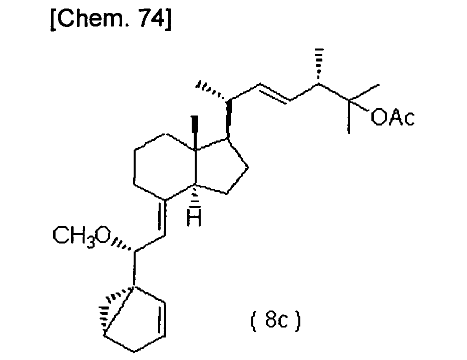

### Physical properties of compound (8c)

¹H-NMR (MeOD) δ (ppm) : 5.90(td,1H,J=2 and 5Hz,H10),5.39(brd,1H,J=5Hz,H1),5.30(m,2H,H22 and H23),4.87(d,1H,J=10Hz,H7),3.97(d,1H,J=10Hz,H6),3.25(s,3H,OMe),2.72(m,1H,H 24), 2.61(dd,1H,J=5 and 13Hz,H9a),2.55(m,1H,H2a),2.27(brd,1H,J=18Hz,H2b), 2.03(m,3H), 1.93(s,3H,Ac),1.72(m,3H),1.47(m,3H),1.39(s,3H,H26), 1.38(s,3H,H27), 1.32(m,4H), 1.03(m,1H,H4a),1.03(d,3H,J=7Hz,H21), 0.98(d,3H,J=7Hz,H28), 0.55(s,3H,H18), 0.15(t,1H,J=4Hz,H4b)

¹³C-NMR (MeOD) δ (ppm) : 172.45(Ac-CO),144.26(C8),139.39(C22), 135.38(C10), 130.01(C23), 128.95(C1),121.07(C7), 86.08(C25),80.18(C6), 57.67(C17),57.04(C14), 56.10(OMe),46.62(C13),46.43(C24), 41.78(C20), 41.60(C12),41.29(C5), 36.92(C2),30.50(C9),28.93(C16),25.13(C11), 24.30(C26),23.57(C27), 23.04(C15),22.49(C4),22.39(Ac-Me), 21.43(C21), 19.98(C3), 15.73(C28), 12.49(C18)
ESI-MS : 477.34[M+Na]⁺

### Example 20 (Embodiment): Synthesis of compound (5d)

To a pyridine (45 mL) solution of the 25-triethylsilyl (TES) derivative (4d) (2.57 g, 4.75 mmol) of compound (4a) was added dropwise and with ice cooling a pyridine (6 mL) solution of osmium tetraoxide (1.20 g, 4.75 mmol). The reaction solution was stirred at room temperature for 2 hours. The reaction solution was concentrated and then extracted with ethyl acetate (150 mL x 3 times). The organic layer was successively washed with 10 percent sodium hydrogensulfite aqueous solution (100 mL), 1 mol/L hydrochloric acid (100 mL x 3 times), 5 percent sodium hydrogencarbonate solution (100 mL), and saturated sodium chloride aqueous solution (100 mL). The organic layer was dried over anhydrous sodium sulfate, filtered, and concentrated, yielding a crude compound (5d) (3.06 g).

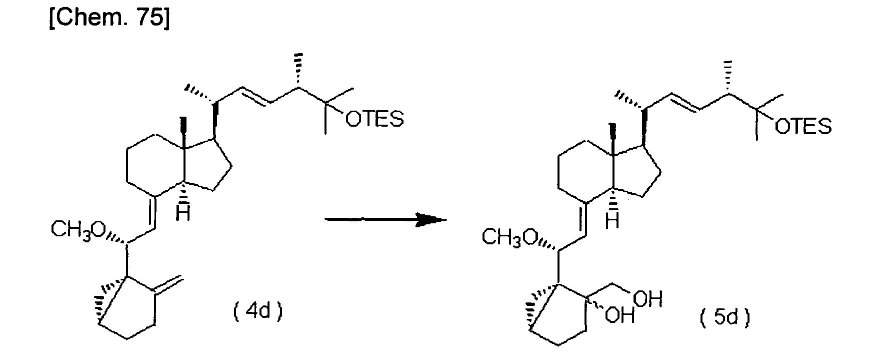

### Example 21 (Embodiment): Synthesis of compound (6d)

Methanol (50 mL) was added to a portion (1.5 g) of the crude compound (5d) obtained in Example 20. A solution of sodium periodate (3.94 g, 7.6 mmol) in purified water (20 mL) was added dropwise at -10°C. This produced a gray, clay-like substance. Methanol (100 mL) was added and the mixture was stirred overnight at room temperature. The reaction solution was filtered through a glass filter coated with cerite, after which the organic solvent was removed. The residue was extracted with ethyl acetate (100 mL x 3 times). The organic layer obtained was successively washed with 5 percent sodium hydrogencarbonate solution (150 mL), purified water (150 mL), and saturated brine (150 mL). The organic layer was dried over anhydrous sodium sulfate, filtered, and concentrated. The residue was separated and purified by silica gel column chromatography (silica gel 60N, spherical, neutral, 63-210 µm, Kanto Chemical Co., Inc., n-hexane/ethyl acetate = 9/1 to 4/1), yielding the compound (6d) (128 mg, a five step total yield from 25-hydroxyvitamin D₂ of 9 percent) denoted below in the form of a colorless viscous liquid.

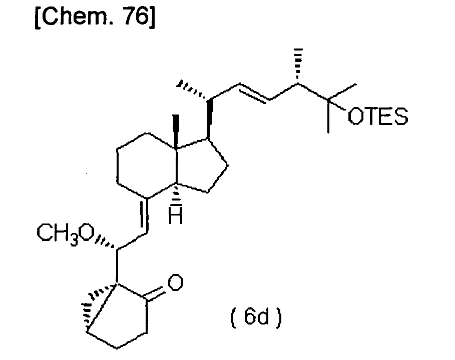

### Physical properties of compound (6d)

¹H-NMR (MeOD) δ (ppm) : 5.36(dd,1H,J=8 and 15Hz,H22orH23), 5.24(dd,1H,J=8 and 15Hz,H22orH23), 4.63(m,2H,H6 and H7),3.18(s,3H,OM e),2.69(m,1H), 2.21(m,2H), 2.06(m,7H),1.73(m,3H),1.55(m,1H), 1.46(m,2H),1.32(m,4H),1.19(s,3H,H26), 1.14(s,3H,H27),1.04(d,3H,J=7Hz,H21), 0.97(neart,13H,CH3,H4a and H28), 0.60(q,6H,J=8Hz,CH2),0.57(s,3H,H18)

### Example 22 (Embodiment): Synthesis of compound (7d)

Lithium hexamethyldisilazide 1.6 mol/L in THF (588 µL, 0.59 mmol) was added to dimethoxyethane (0.6 mL) at -75°C. A dimethoxyethane (0.5 mL) solution of compound (6d) (128 mg, 0.24 mmol) was added dropwise and the mixture was stirred for 0.5 hour at -75°C. To this reaction was added N-phenylbis(trifluoromethanesulfonimide) (210 mg, 0.59 mmol). The reaction temperature was immediately lowered to -50°C. The temperature was then raised from that temperature to room temperature over 15 hours with stirring. The reaction solution was concentrated and then diluted with ethyl acetate (5 mL). The organic layer was successively washed with 0.5 mol/L hydrochloric acid (3 mL), 5 percent sodium hydrogencarbonate solution (3 mL), and saturated sodium chloride solution (3 mL). The organic layer was dried over anhydrous sodium sulfate, filtered, and concentrated. The residue was separated and purified by preparative TLC (Merck silica gel 60 F254 1.05744, n-hexane/ethyl acetate = 9/1), yielding the compound (7d) (83 mg, yield of 52 percent) denoted below in the form of a colorless, viscous liquid.

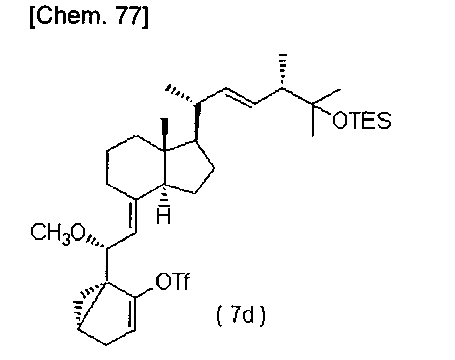

### Physical properties of compound (7d)

¹H-NMR (MeOD) δ (ppm) : 5.35(m,2H,H22 and H1),5.23(dd,1H,J=8 and 15Hz,,H23), 4.65(d,1 H,J=9Hz,H7),4.38(d,1 H,J=9Hz,H6),3.24(s,3H,OMe), 2.60(m,2H),2.35(dd,1H,J=3 and 18Hz,H2a),2.04(m,4H),1.76(m,4H), 1.48(m,3H),1.35(m,3H),1.19(s,3H,H26),114(s,3H,H27),1.09(dd, H,J=5 and 8Hz,H4a), 1.03(d,3H,J=7Hz,H28),0.97(comp,12H,CH3 and H28),0.59(comp,9H,CH2 and H18), 0.53(t,1H,J=5Hz,H4b)

¹³C-NMR (MeOD) δ (ppm) : 153.88(C10),147.27(C8),137.71(C23),132.14(C22),118.07(C7),114.97(C1), 76.50(C25orC6),76.47(C25orC6),57.83(C17),57.04(C14),56.44(OMe),50.43(C24 ), 46.52(C13),41.79(C20),41.38(C12),36.90(C5),31.03(C2),30.32(C9),29.20(C26), 28.90(C11),27.81(C27),24.73(C16),23.07(C15),21.42(C28),20.08(C4),18.55(C3), 15.88(C21),12.65(C18),7.85(TES-CH2),7.54(TES-CH3).
ESI-MS : 697.44[M+Na]⁺ ,709.46[M+Cl]⁻

### Example 23 (Embodiment) Synthesis of compound (8d)

The compound (7d) (83 mg) obtained in Example 22, triphenylphosphine (9.7 mg, 0.037 mmol), and palladium diacetate (5.6 mg, 0.025 mmol) were dissolved in dimethylformamide (0.5 mL). Tributylamine (88 µL, 0.37 mmol) and formic acid (9 µL, 0.25 mmol) were successively added dropwise at room temperature, and the mixture was stirred at 60°C for 1 hour. The reaction solution was allowed to cool, and then concentrated. The residue was separated and purified by preparative TLC (Merck silica gel 60 F254 1.05744, n-hexane/ethyl acetate = 9/1), yielding the compound (8d) (35 mg, yield of 54 percent) denoted below in the form of a colorless, viscous liquid.

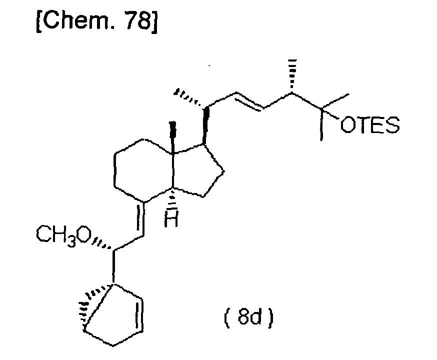

### Physical properties of compound (8d)

¹H-NMR (MeOD) δ (ppm) : 5.91(neard,H10),5.39(neard,1H,H1),5.36(dd,J=8 and 15Hz,1H,H22), 5.23(dd,J=8 and 15Hz,1H,H23), 4.88(neard,1H,H7), 3.98(d,1H,J=9Hz,H6), 3.26(s,3H,OMe),2.57(m,2H),2.27(brd,1H,J=18Hz,H2a), 2.01(m,4H),1.72(m,3H), 1.44(m,3H),1.34(m,4H),1.19(s,3H,H26),1.14(s,3H,H27), 1.11 (comp,16H), 0.60(q,6H,J=8Hz,CH2),0.55(s,3H,H18),0.17(t,1H,J=4Hz,H4a)

¹³C-NMR (MeOD) δ (ppm) : 144.31(C8),137.78(C23),135.38(C10),132.06(C22),129.00(C1),121.03(C7), 80.19(C6),76.46(C25),57.87(C17),57.06(C14),56.10(OMe),50.42(C24), 46.60(C13), 41.80(C20), 41.62(C12),41.28(C5),36.92(C2),30.50(C9), 29.19(C27),28.95(C16),27.79(C26), 25.13(C15),23.03(C11),22.48(C4), 21.43(C21),19.98(C3),15.87(TES-CH3),12.48(C18), 7.84(TES-CH2),7.60(C28)
ESI-MS : 549.48[M+Na]⁺

### Example 24 (Embodiment): Synthesis of compound (5e)

Pyridine (5.0 mL) was added to crude compound (4e) (1.12 g) at 0°C. A pyridine (5.0 mL) solution of osmium tetraoxide (0.61 g, 2.4 mmol) was added dropwise and the mixture was stirred at room temperature for 1 hour. To the solution was added 100 mL of a 10 percent sodium hydrogensulfite aqueous solution at 0°C and the mixture was stirred for 1 hour at room temperature, at which point the reaction ended. The reaction solution was extracted with ethyl acetate (50 mL x 3 times). The organic layer was successively washed with 2 mol/L hydrochloric acid (20 mL x 3 times), water (20 mL), saturated sodium hydrogencarbonate aqueous solution (20 mL), and saturated sodium chloride aqueous solution (20 mL). The organic layer was dried over anhydrous sodium sulfate, filtered, and concentrated, yielding the crude compound (5e) (1.08 g) denoted below in the form of a brown amorphous product.

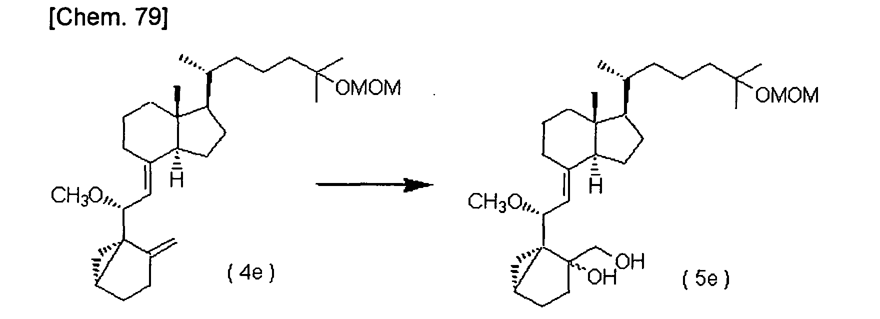

### Physical properties of compound (5e)

¹H-NMR (MeOD) δ (ppm) : 4.90(d,1H,J=9Hz,H6),4.68(s,2H,MOM-CH2), 4.58(d,1H,J=9Hz,H7), 3.65(d,1H,J=11Hz,H19a),3.58(d,1H,J=11Hz,H19b), 3.33(s,3H,MOM-CH3),3.20(s,3H,OMe), 2.70(m,1H,H9a), 2.18(m,3H), 1.93(m,1H,H16a), 1.70(m,2H),1.63(dd,1H,J=7 and 12Hz,H2b),1.58-1.20(m,15H),1.20(s,6H,H26 and H27),1.07(m,1H,H22b), 0.96(d,3H,J=6Hz,H21),0.59(s,3H,H18),0.51(dd,1H,J=5 and 8Hz, H4a),0.38(t,1H,J=5Hz,H4b)

¹³C-NMR (MeOD) δ (ppm) : 146.88(C8),119.17(C7),92.01(MOM-CH2), 85.07(C10), 78.76(C6),77.65(C25),68.34(C19),58.01(C17),56.99(C14), 55.89(OMe), 55.45(MOM-CH3),46.64(C13),43.38(C24),41.59(C12),37.67(C22), 37.46(C20), 36.48(C5), 33.76(C1),30.20(C9),28.70(C16),26.81(C26),26.73(C27), 25.27(C2), 24.46(C11), 23.34(C15),21.62(C23),21.33(C3),19.38(C21), 12.61 (C18),11.85(C4)
ESI-MS : 515.47[M+Na]⁺, 527.50[M+Cl]⁻

### Example 25 (Embodiment): Synthesis of compound (6e)

The crude compound (5e) was dissolved in methanol (25 mL) at 0°C. An aqueous solution (10 mL) of sodium periodate (151 g, 7.0 mmol) was added dropwise and the mixture was stirred at 0°C for 1 hour, resulting in termination of the reaction. The reaction solution was concentrated, diluted with water (10 mL), and extracted with ethyl acetate (50 mL x 2 times). The organic layer was successively washed with water (20 mL), 1 mol/L hydrochloric acid (20 mL), saturated sodium hydrogencarbonate aqueous solution (20 mL), and saturated sodium chloride aqueous solution (20 mL). The organic layer was dried over anhydrous sodium sulfate, filtered, and concentrated. The residue was separated and purified by silica gel column chromatography (silica gel 60N (spherical, neutral, 63-210 µm, Kanto Chemical Co., Inc.) 22 g, n-hexane/ethyl acetate = 6/1), yielding the compound (6e) (453.8 mg, combined 5 step yield from 25-hydroxyvitamin D₂ of 38 percent) denoted below in the form of a brown oil.

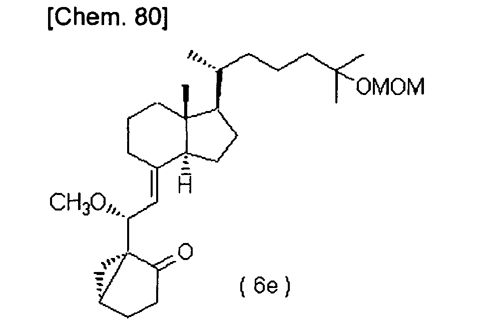

### Physical properties of compound (6e)

¹H-NMR (MeOD) δ (ppm) : 4.68(s,2H,MOM-CH2), 4.65(d,1H,J=9Hz,H6), 4.62(d,1H,J=9Hz,H7), 3.33(s,3H,MOM-CH3),3.18(s,3H,OMe), 2.63(m,1H,H9a),2.19(m,2H), 2.06(m,4H),1.95(m,2H),1.72(m,2H),1.55-1.23(m,13H), 1.02(s,6H,H26 and H27),1.03(m,2H),0.96(d,3H,J=6Hz,H21),0.57(s,3H,H18)

¹³C-NMR (MeOD) δ (ppm) : 216.71(C10),146.89(C8),118.22(C7), 91.97(MOM-CH2),77.62(C25),74.70(C6),57.96(C17),56.99(C14),56.45(OMe), 55.41 (MOM-CH3),46.64(C13),43.34(C24),41.95(C5),41.57(C12),37.64(C22), 37.42(C20), 33.90(C1),30.32(C9),28.68(C16),26.77(C26),26.69(C27),25.73(C3), 24.57(C11), 23.16(C15),22.47(C2),21.58(C23),19.35(C21),17.97(C4),12.42(C18)
ESI-MS : 483.40[M+Na]⁺,495.51[M+Cl]⁻

### Example 26 (Embodiment): Synthesis of compound (7e)

Lithium hexamethyldisilazide 1.6 mol/L in THF (1.07 mL, 1.7 mmol) was added to dimethoxyethane (2.0 mL) at -75°C, a dimethoxyethane (4.0 mL) solution of compound (6e) (315.9 mg, 0.68 mmol) was added dropwise, and the mixture was stirred for 1.5 hours at -75°C. To this solution was added N-phenylbis(trifluoromethanesulfonimide) (612 mg, 1.7 mmol) and the mixture was stirred at -75 to 20°C for 16 hours, resulting in termination of the reaction. The reaction solution was concentrated, diluted with water (10 mL), and extracted with ethyl acetate (20 mL x 3 times). The organic layer was successively washed with 1 mol/L hydrochloric acid (3 mL), saturated sodium hydrogencarbonate aqueous solution (3 mL), and saturated sodium chloride aqueous solution (3 mL). The organic layer was dried over anhydrous sodium sulfate, filtered, and concentrated, yielding the crude compound (7e) (733.9 mg) denoted below in the form of a brown, amorphous product.

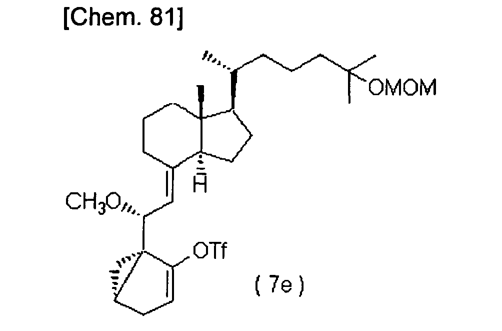

### Physical properties of compound (7e)

¹H-NMR (MeOD) δ (ppm) : 5.33(d,1H,J=2Hz,H1),4.68(s,2H,MOM-CH2), 4.66(d,1H,J=9Hz,H7), 4.37(d,1 H,J=9Hz,H6),3.33(s,3H,MOM-CH3), 3.24(s,3H,OMe), 2.59(m,2H),2.34(dd,1H,J=2 and 17Hz,H2b), 2.02(m,2H), 1.93(m,1H,H16a), 1.74(m,3H), 1.51-1.25(m,12H), 1.20(s,6H,H26 and H27), 1.09(dd,1H,J=4 and 8Hz,H4a), 1.07(m,1H,H22b), 0.96(d,3H,J=6Hz,H21), 0.56(s,3H,H18),0.52(t,1H,J=4Hz,H4b)

¹³C-NMR (MeOD) δ (ppm) : 153.92(C10),147.33(C8),118.10(C7),115.01(C1),92.01(MOM-CH2),77.64(C25), 76.57(C6),58.00(C17),57.02(C14),56.47(OMe),55.44(MOM-CH3),46.66(C13), 43.38(C24),41.56(C12),37.67(C22),37.47(C20),36.94(C5),31.06(C2),30.35(C9), 28.70(C16),26.80(C26),26.73(C27),24.79(C11),23.16(C15),21.62(C23),20.13(C4 ), 19.38(C21),18.59(C3),12.40(C18)
ESI-MS : 615.37[M+Na]⁺

### Example 27 (Embodiment): Synthesis of compound (8e)

The crude compound (7e) (733.9 mg) obtained in Example 26, triphenylsulfine (34.7 mg, 0.13 mmol), and palladium diacetate (14.6 mg, 0.005 mmol) were dissolved in dimethylamide (5 mL). Tributylamine (472 µL, 1.98 mmol) and formic acid (50 µL, 1.32 mmol) were successively added dropwise at room temperature, and the mixture was stirred at 60°C for 50 minutes, resulting in termination of the reaction. The reaction solution was diluted with water (10 mL) and extracted with ethyl acetate (30 mL x 3 times). The organic layer was successively washed with 1 mol/L hydrochloric acid (4 mL), saturated sodium hydrogencarbonate aqueous solution (4 mL), and saturated sodium chloride aqueous solution (4 mL). The organic layer was dried over anhydrous sodium sulfate, filtered, and concentrated. The residue was separated and purified by silica gel column chromatography (silica gel 60N (spherical, neutral, 63-210 µm, Kanto Chemical Co., Inc.) 7.5 g, n-hexane/ethyl acetate = 6/1), yielding the compound (8e) (98.7 mg, combined 2 step yield from compound 6e of 32 percent) denoted below in the form of a slightly yellow amorphous product.

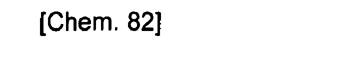

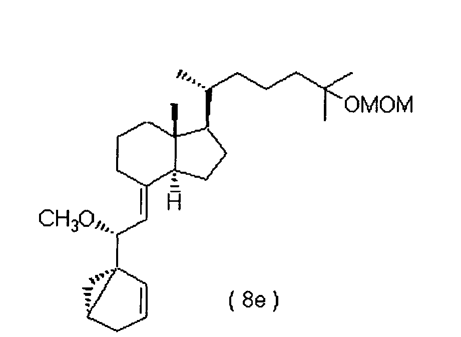

### Physical properties of compound (8e)

¹H-NMR (MeOD) δ (ppm) : 5.90(td,1H,J=2 and 5Hz,H10),5.39(brd,1H,J=5Hz,H1), 4.87(d,1H,J=9Hz,H7),4.67(s,2H,MOM-CH2), 3.97(d,1H,J=9Hz,H6), 3.33(s,3H,MOM-CH3),3.25(s,3H,OMe),2.61 (dd,1H,J=4 and 11 Hz,H9a), 2.54(m,1H,H2a),2.27(brd,1H,J=17Hz,H2b),2.01(m,2H), 1.92(m,1H,H16a), 1.70(m,2H),1.51-1.22(m,12H),1.23(s,6H,H26 and H27),1.03(m,1H,H22b), 1.03(dd,1H,J=4 and 8Hz,H4a),0.96(d,3H,J=6Hz,H21), 0.58(s,3H,H18), 0.16(t,1H,J=4Hz,H4b)

¹³C-NMR (MeOD) δ (ppm) : 144.38(C8),135.38(C10),128.95(C1),121.00(C7),91.97(MOM-CH2),80.20(C6), 77.62(C25),58.01(C17),57.01(C14),56.10(OMe),55.42(MOM-CH3),46.71(C13), 43.35(C24),41.78(C12),41.29(C5),37.67(C2),37.46(C20),36.92(C22),30.51 (C9), 28.74(C16),26.77(C26),26.70(C27),25.16(C11),23.09(C15),22.49(C4),21.59(C23 ), 19.97(C3),19.38(C21),12.20(C18)
ESI-MS : 467.45[M+Na]⁺

### Example 28 (Embodiment): Synthesis of compound (9c)

The compound (8e) (33 mg, 0.074 mmol) obtained in Example 27 was dissolved in tetrahydrofuran (1 mL), 9-borabicyclo[3.3.1]nonane 0.5 mol/L in THF (350 µL, 0.17 mmol) was added dropwise at 0°C, and the mixture was stirred at room temperature for 2.5 hours, resulting in disappearance of the starting material. To the reaction solution was added a mixture (1:1) (500 µL) of 30 percent hydrogen peroxide aqueous solution and 3 mol/L sodium hydroxide aqueous solution and the mixture was stirred at room temperature for 15 hours. The reaction solution was diluted with water (10 mL) and extracted with ethyl acetate (15 mL x 3 times). The organic layer was successively washed with 1 mol/L hydrochloric acid (2 mL), saturated sodium hydrogencarbonate aqueous solution (2 mL) and saturated sodium chloride aqueous solution (2 mL). The organic layer was dried over anhydrous sodium sulfate, filtered, and concentrated. The residue was separated and purified by preparative TLC (Merck silica gel 60 F254 1.05744, n-hexane/ethyl acetate = 3/1), yielding the compound (9c) (10.0 mg, yield of 29 percent) denoted below in the form of a colorless oil.

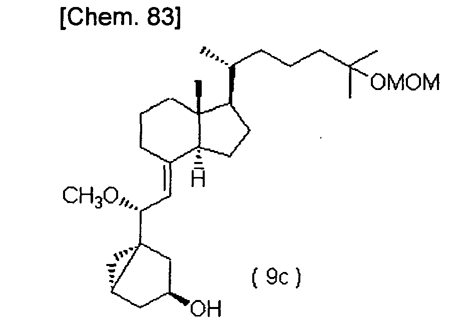

### Physical properties of compound (9c)

¹H-NMR (MeOD) δ (ppm) : 4.95(d,1H,J=9Hz,H7),4.69(s,2H,MOM-CH2), 3.93(m,1H,H1β), 3.89(d,1H,J=9Hz,H6), 3.33(s,3H,MOM-CH3),3.21(s,3H,OMe), 2.63(m,1H,H9a),2.04(m,4H),1.93(m,1H,H16a),1.75(dd,1H,J=8 and 13Hz,H10b),1.71-1.24(m,14H),1.20(s,6H,H26 and H27), 1.13(m,1H,H3),1.05(m,1H,H22 b), 0.97(d,3H,J=7Hz,H21), 0.62(s,3H,H18),0.55(dd,1H,J=5 and 9Hz,H4a), 0.38(t,1H,J=5Hz,H4b)

¹³C-NMR (MeOD) δ (ppm) : 144.92(C8),120.33(C7),91.97(MOM-CH2), 81.20(C6),77.62(C25), 71.24(C1),58.01(C17),57.10(C14),56.17(OMe), 55.41(MOM-CH3), 46.67(C13), 43.35(C24),41.75(C12),37.85(C10),37.67(C2),37.46(C20),37.27(C22),31.53(C5), 30.52(C9),28.76(C16),26.77(C26),26.70(C27),25.00(C11),23.16(C15), 21.60(C23),19.46(C3),19.39(C21),14.79(C4),12.52(C18)
ESI-MS : 485.43[M+Na]⁺, 497.30[M+Cl]⁻

### Example 29 (Embodiment): Synthesis of compounds (10e and 10f)

The compound (9c) (6.4 mg, 0.013 mmol) obtained in Example 28 was dissolved in acetic acid (0.2 mL) and stirred at room temperature for 1 hour, resulting in termination of the reaction. The reaction solution was diluted with ice and saturated sodium hydrogencarbonate (5 mL) and extracted with ethyl acetate (5 mL x 3 times). The organic layer was washed with saturated sodium chloride aqueous solution (2 mL). The organic layer was dried over sodium sulfate, filtered, and concentrated, yielding a mixture (7.0 mg) of crude compounds (10e and 10f).

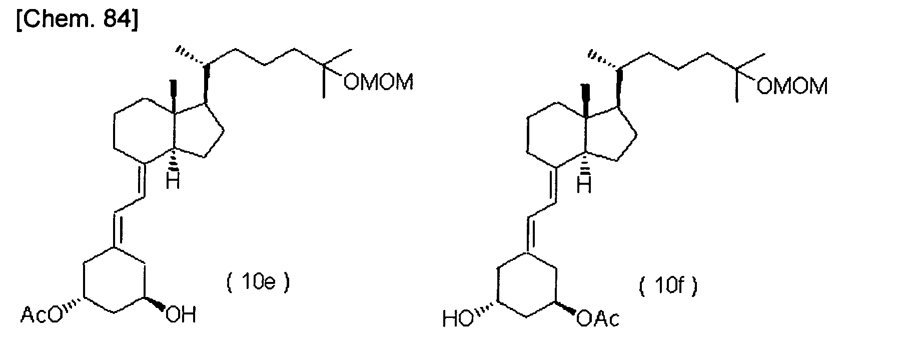

### Example 30 (Reference Example): Synthesis of compound (11 b)

To the mixture (7.0 mg) of compounds (10e and 10f) obtained in Example 29 was added a 10 percent potassium hydroxide ethanol solution (0.3 mL) at 0°C and the mixture was stirred at room temperature for 1 hour, resulting in termination of the reaction. The reaction solution was concentrated and the residue was separated and purified by preparative TLC (Merck silica gel 60 F254 1.05744, n-hexane/ethyl acetate = 1/1), yielding the compound (11 b) (4.5 mg, yield of 73 percent) denoted below in the form of a colorless oil.

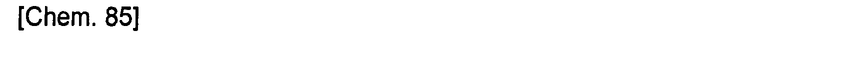

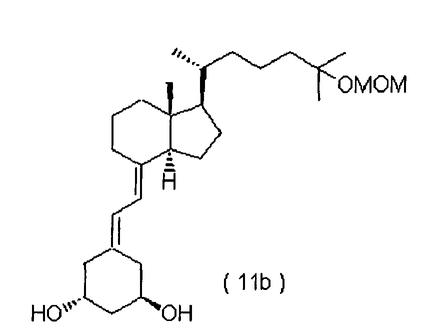

### Physical properties of compound (11 b)

¹H-NMR (MeOD) δ (ppm) : 6.21(d,1H,J=11Hz,H6),5.88(d,1H,J=11Hz,H7),4.68(s,2H,MOM-CH2), 4.03(m,1H,H1β),3.98(m,1H,H3a),3.33(s,3H,MOM-CH3),2.83(m,1H,H9a), 2.59(dd,1H,J=4 and 13Hz,H4a),2.41(dd,1H,J=4 and 13Hz,H10a),2.20(dd,1H,J=7 and 13Hz,H4b),2.16(dd,1H,J=6 and 13Hz,H10b), 2.02(m,2H), 1.93(m,1H,H16a), 1.82(m,1H,H2a),1.77(m,1H,H2b),1.72-1.24(m,14H),1.20(s,6H,H26 and H27), 1.07(m,1H,H22b),0.96(d,3H,J=6Hz,H21),0.57(s,3H,H18)

¹³C-NMR (MeOD) δ (ppm) : 142.11(C8),133.90(C5),123.45(C6),117.16(C7),91.97(MOM-CH2), 77.63(C25), 68.00(C1),67.72(C3),58.03(C17),57.54(C14),55.42(MOM-CH3),46.82(C13), 45.42(C10),43.35(C24),42.70(C2),41.94(C12),37.68(C4 and C22),37.49(C20), 29.85(C9),28.78(C16),26.78(C26),26.70(C27),24.53(C11),23.30(C15),21.60(C23 ), 19.40(C21),12.43(C18)
ESI-MS : 471.45[M+Na]⁺, 483.45[M+Cl]⁻

### Example 31 (Reference Example): Synthesis of 1,25-dihydroxy-19-norvitamin D₃ (12b)

The compound (11 b) (5.1 mg, 0.011 mmol) obtained in Embodiment 27 was dissolved in a mixture (1:3) (0.5 mL) of tetrahydrofuran and methanol. Camphor sulphonic acid (6.5 mg, 0.028 mmol) was added at 0°C, and the mixture was stirred at room temperature for 5.5 hours, resulting in termination of the reaction. The reaction solution was diluted with saturated sodium hydrogencarbonate (3 mL) and extracted with ethyl acetate (5 mL x 3 times). The organic layer was washed with saturated sodium chloride aqueous solution (2 mL). The organic layer was dried over anhydrous sodium sulfate, filtered, and concentrated. The residue was separated and purified by preparative TLC (Merck silica gel 60 F254 1.05715, chloroform/methanol = 10/1), yielding 1,25-dihydroxy-19-norvitamin D₃ (12b) (3.0 mg, yield of 67 percent) in the form of a white powder.

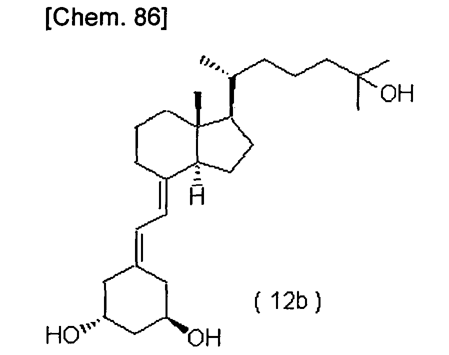

### Physical properties of 1,25-dihydroxy-19-norvitamin D₃ (12b)

¹H-NMR (MeOD) δ (ppm) : 6.21(d,1H,J=11Hz,H6),5.88(d,1H,J=11Hz,H7),4.03(m,1H,H1β),3.98(m,1H,H3a), 2.83(m,1H,H9a),2.59(dd,1H,J=4 and 13Hz,H4a),2.41(dd,1H,J=4 and 13Hz,H10a),2.20(dd,1H,J=7 and 13Hz,H4b),2.16(dd,1H,J=6 and 13Hz,H10b), 2.02(m,2H),1.93(m,1H,H16a),1.83(m,1H,H2a),1.77(m,1H,H2b), 1.72-1.23(m,14H), 1.17(s,6H,H26 and H27),1.07(m,1H,H22b), 0.96(d,3H,J=6Hz,H21),0.57(s,3H,H18)

¹³C-NMR (MeOD) δ (ppm) : 142.15(C8),133.93(C5),123.49(C6),117.19(C7),71.53(C25),68.04(C1),67.75(C3), 58.06(C17),57.57(C14),48.54(C13),45.45(C10),43.35(C24),42.74(C2), 41.98(C12), 37.81(C4orC22),37.70(C4orC22),37.52(C20),29.88(C9),29.31(C26), 29.18(C27), 28.81(C16),24.57(C11),23.34(C15),21.96(C23),19.43(C21), 19.46(C18)
ESI-MS : 427.38[M+Na]⁺, 439.39[M+Cl]⁻

## Claims

1. A process of producing the 1-hydroxy-19-norvitamin D derivative denoted by formula (X): 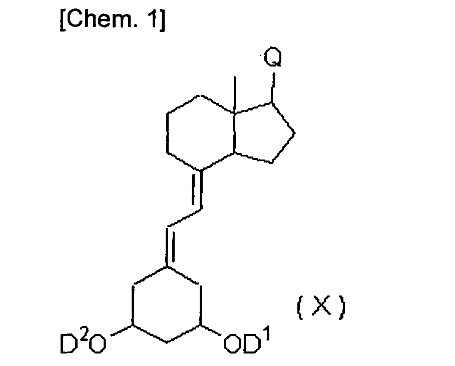 (wherein Q denotes a hydrogen atom, optionally substituted C₁ to C₅ alkyl group, or (Q-I) 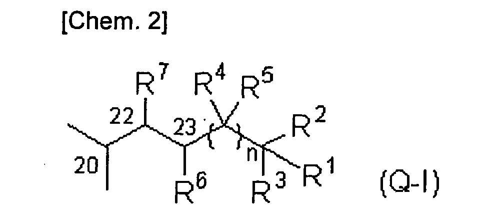 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combined to denote a carbon-carbon double bond; n denotes an integer of 1 to 5; and one of the atoms at position 20, 22 or 23 is optionally substituted with an oxygen, sulfur or nitrogen atom),
and each of D¹ and D² denote hydrogen atoms or C₁ to C₇ acyloxy groups),
**characterized by**:
a) reacting the cyclovitamin D derivative denoted by formula (IV): 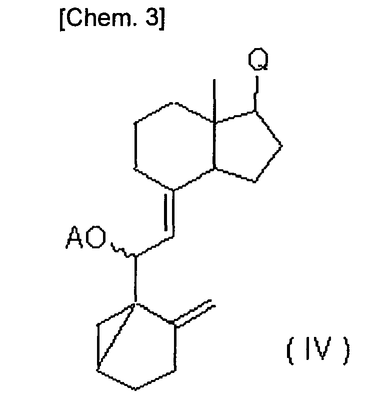 (wherein A denotes a C₁ to C₃ alkyl group and Q is defined as above) with osmium tetraoxide or a permanganate salt in an inert solvent; to produce the 10,19-diolcyclovitamin D derivative denoted by formula (V): 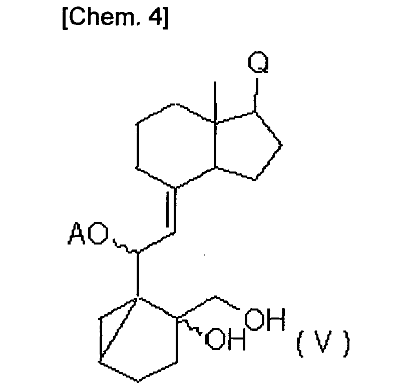 (wherein Q and A are defined as above);
b) reacting the 10,19-diolcyclovitamin D derivative of formula (V) thus obtained with a perhalogenate salt or with lead tetraacetate in an inert solvent to produce the 10-oxocyclovitamin D derivative denoted by general formula (VI): 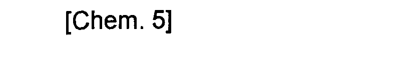 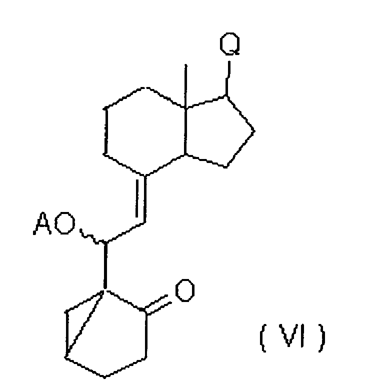 (wherein Q and A are defined as above);
c) reacting the 10-oxocyclovitamin D derivative of formula (VI) thus obtained with PY₅ (wherein Y denotes a halogen atom) or a base and R⁸SO₂-Z (wherein R⁸ denotes an optionally substituted C₁ to C₆ alkyl group, optionally substituted C₂ to C₆ unsaturated alkyl group, optionally substituted C₆ to C₁₄ aryl group or optionally substituted C₇ to C₂₂ aralkyl group; Z denotes a halogen, R⁸SO₂O-(wherein R⁸ is defined as above) or R⁸SO₂(WN- (wherein R⁸ is defined as above, W denotes an optionally substituted C₁ to C₆ alkyl group, optionally substituted C₂ to C₆ unsaturated alkyl group, optionally substituted C₆ to C₁₄ aryl group, or optionally substituted C₇ to C₂₂ aralkyl group)) in an inert solvent to produce the cyclovitamin D derivative denoted by formula (VII): 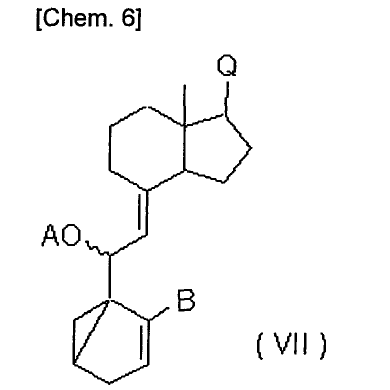 (wherein Q and A are defined as above, D denotes a halogen atom or R⁸SO₂O-(wherein R⁸ is defined as above));
d) reacting the cyclovitamin D derivative of formula (VII) thus obtained with a hydrogen source , an amine and noble metal catalyst in an inert solvent to produce the 1,10-olefincyclovitamin D derivative denoted by formula (VIII): 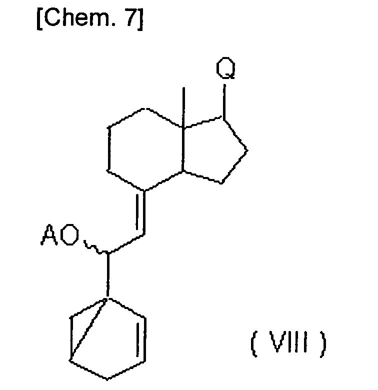 (wherein Q and A are defined as above);
e) reacting the 1,10-olefincyclovitamin D derivative denoted by formula (VIII) thus obtained with a hydroboronating agent in an inert solvent to obtain the 1-hydroxycyclovitamin D derivative denoted by general formula (IX): 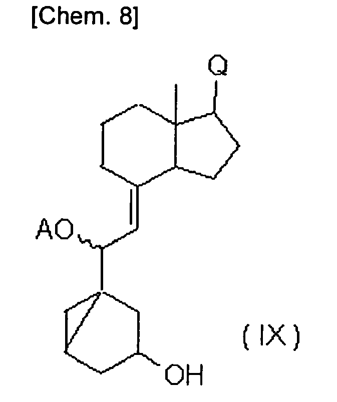 (wherein Q and A are defined as above); and
f) solvolysing the 1-hydroxycyclovitamin D derivative of formula (IX) thus obtained in the presence of an optionally substituted C₁ to C₁₁ organic acid.

2. A process of producing the cyclovitamin D derivative denoted by formula (VII): 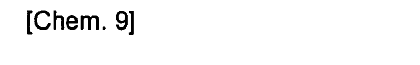 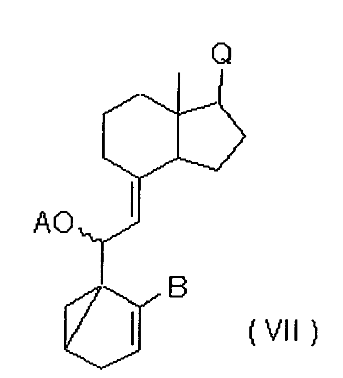 (wherein Q denotes a hydrogen atom, optionally substituted C₁ to C₅ alkyl group, or
(Q-I): 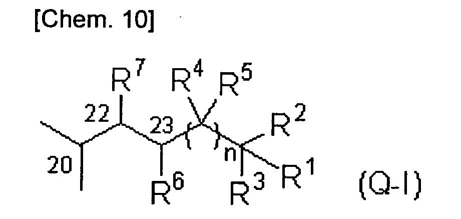 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom),
A denotes a C₁ to C₃ alkyl group, and
B denotes a halogen atom or R⁸SO₂O- (wherein R⁸ denotes an optionally substituted C₁ to C₆ alkyl group, optionally substituted C₂ to C₆ unsaturated alkyl group, optionally substituted C₆ to C₁₄ aryl group or optionally substituted C₇ to C₂₂ aralkyl group)), **characterized by**:
reacting the 10-oxocyclovitamin D derivative denoted by formula (VI): 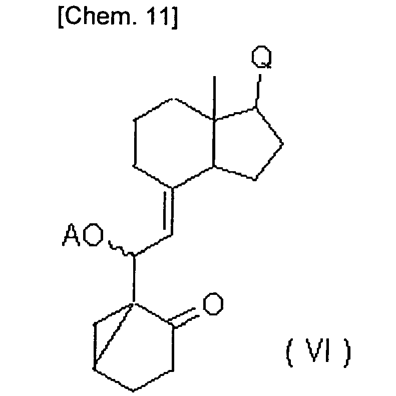 (wherein Q and A are defined as above)
with PY₅ (wherein Y denotes a halogen atom) or a base and R⁸SO₂-Z (wherein R⁸ is defined as above, Z denotes a halogen, R⁸SO₂O- (wherein R⁸ is defined as above), or R⁸SO₂(W)N- (wherein R⁸ is defined as above, W denotes an optionally substituted C₁ to C₆ alkyl group, optionally substituted C₂ to C₆ unsaturated alkyl group, optionally substituted C₆ to C₁₄ aryl group or optionally substituted C₇ to C₂₂ aralkyl group)) in an inert solvent.

3. The process of producing a cyclovitamin D derivative according to Claim 2, wherein Q denotes formula (Q-I): 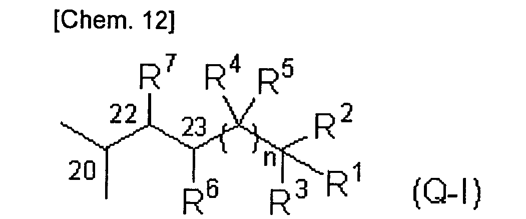 (wherein R¹ denotes a hydrogen atom, hydroxyl group or -OR^{1"}, where R^{1"} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group, or propyl group; each of R⁴ and R⁵ denotes a hydrogen atom or methyl group; each of R⁶ and R⁷ denotes a hydrogen atom ,or R⁶ and R⁷ combine to form a carbon-carbon double bond; n denotes an integer of 1 to 3; and one of the carbons at position 20, 22 or 23 is optionally substituted with an oxygen, sulfur, or nitrogen atom);
A denotes a methyl group, and
B denotes R^{8'}SO₂O- (wherein R^{8'} denotes an optionally substituted C₁ to C₄ alkyl group, optionally substituted C₆ or C₇ aryl group or optionally substituted C₇ or C₈ aralkyl group).

4. The process of producing a cyclovitamin D derivative according to Claim 2, wherein Q denotes formula (Q-II): 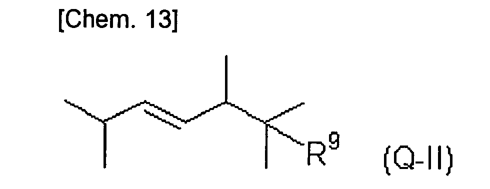 (wherein R⁹ denotes a hydrogen atom, acetoxy group, hydroxyl group, trimethylsilyloxy group, triethylsilyloxy group, methoxymethyloxy group or benzyloxymethyloxy group)
or formula (Q-III) 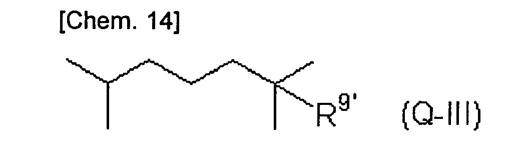 (wherein R^{9'} denotes a hydrogen atom, acetoxy group, trimethylsilyloxy group, triethylsilyloxy group, methoxymethyloxy group or benzyloxymethyloxy group);
A denotes a methyl group, and
B denotes a trifluoromethanesulfonyloxy group.

5. A process of producing a 1,10-olefincyclovitamin D derivative denoted by formula (VIII): 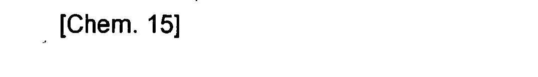 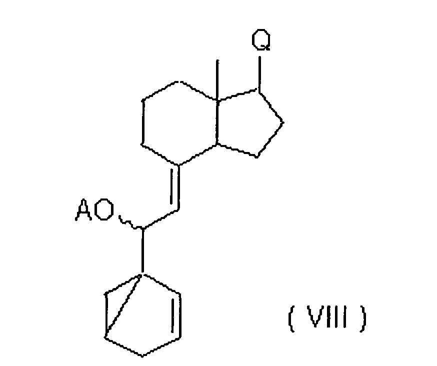 (wherein Q denotes a hydrogen atom, optionally substituted C₁ to C₅ alkyl group, or
(Q-I): 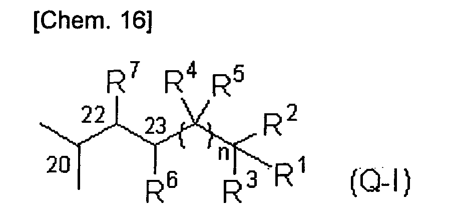 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom),
and A denotes a C₁ to C₃ alkyl group),
**characterized by** reacting the cyclovitamin D derivative denoted by formula (VII): 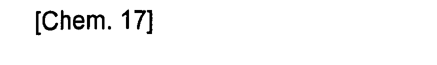 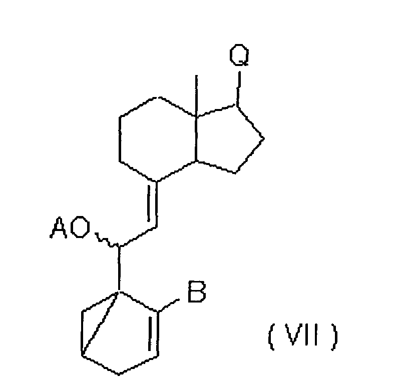 (wherein Q and A are defined as above, B denotes a halogen atom or R⁸SO₂O-(wherein R⁸ denotes an optionally substituted C₁ to C₆ alkyl group, optionally substituted C₂ to C₆ unsaturated alkyl group, optionally substituted C₆ to C₁₄ aryl group or optionally substituted C₇ to C₂₂ aralkyl group)) with an amine, hydrogen source and noble metal catalyst in an inert solvent.

6. The process of producing a 1,10-olefincyclovitamin D derivative according to Claim 5, wherein Q denotes formula (Q-I): 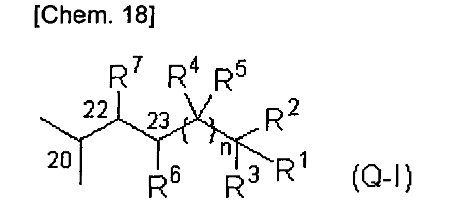 (wherein R¹ denotes a hydrogen atom, hydroxyl group or -OR^{1'}, where R^{1'} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group, or propyl group; each of R⁴ and R⁵ denotes a hydrogen atom or methyl group; each of R⁶ and R⁷ denotes a hydrogen atom, or R⁶ and R⁷ combine to form a carbon-carbon double bond; n denotes an integer of 1 to 3; and one of the carbons at position 20, 22 or 23 is optionally substituted with an oxygen, sulfur or nitrogen atom); and
A denotes a methyl group.

7. The process of producing a 1,10-olefincyclovitamin D derivative according to Claim 5, wherein Q denotes formula (Q-II): 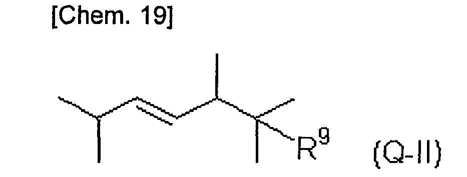 (wherein R⁹ denotes a hydrogen atom, acetoxy group, hydroxyl group, trimethylsilyloxy group, triethylsilyloxy group, methoxymethyloxy group or benzyloxymethyloxy group)
or formula (Q-III) 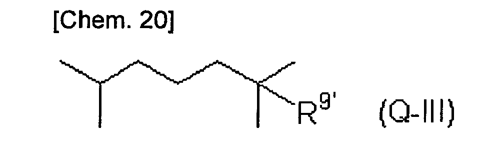 (wherein R^{9'} denotes a hydrogen atom, acetoxy group, trimethylsilyloxy group, triethylsilyloxy group, methoxymethyloxy group or benzyloxymethyloxy group); and
A denotes a methyl group.

8. A process of producing a 1-hydroxy-19-norcyclovitamin D derivative denoted by formula (IX): 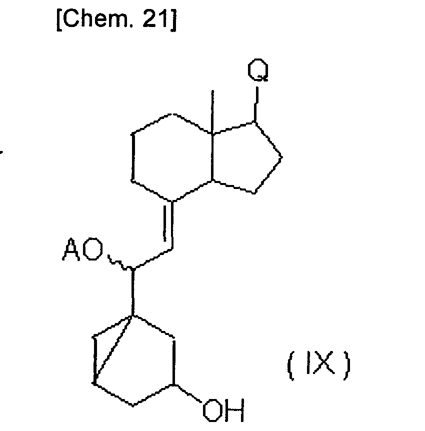 (wherein Q denotes a hydrogen atom, optionally substituted C₁ to C₅ alkyl group, or (Q-I): 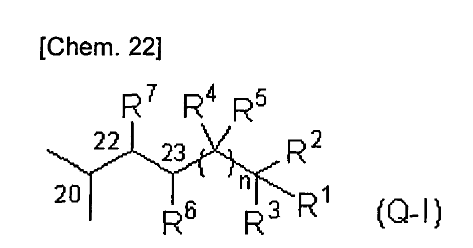 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom), and
A denotes a C₁ to C₃ alkyl group);
**characterized in that** the 1,10-olefincyclovitamin D derivative denoted by general formula (VIII): 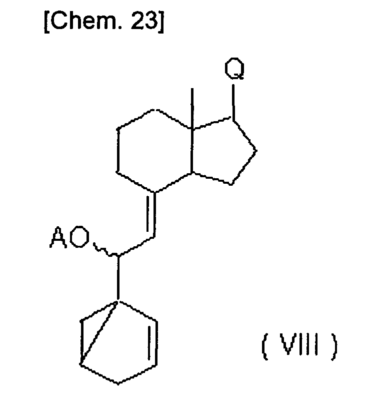 (wherein Q and A are defined as above)
is reacted with a hydroboronating agent in an inert solvent.

9. The process of producing a 1-hydroxy-19-norchlorovitamin D derivative according to Claim 8, wherein Q denotes formula (Q-I): 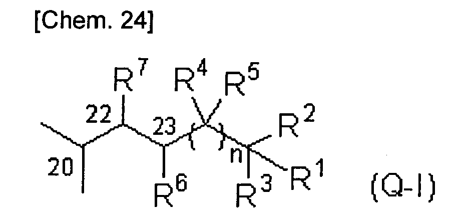 (wherein R¹ denotes a hydrogen atom, hydroxyl group or -OR^{1'}, where R^{1'} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group, or propyl group; each of R⁴ and R⁵ denotes a hydrogen atom or methyl group; each of R⁶ and R⁷ denotes a hydrogen atom, or R⁶ and R⁷ combine to form a carbon-carbon double bond; n denotes an integer of 1 to 3; and one of the carbons at position 20, 22 or 23 is optionally substituted with an oxygen, sulfur or nitrogen atom); and
A denotes a methyl group.

10. The process of producing a 1-hydroxy-19-norchlorovitamin D derivative according to Claim 8, wherein Q denotes formula (Q-II): 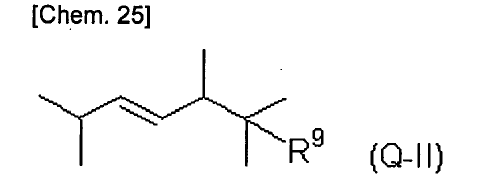 (wherein R⁹ denotes a hydrogen atom, acetoxy group, hydroxyl group, trimethylsilyloxy group, triethylsilyloxy group, methoxymethyloxy group, or benzyloxymethyloxy group)
or formula (Q-III): 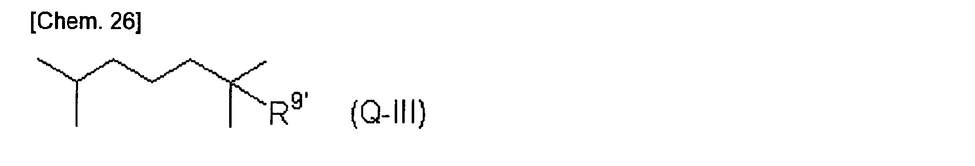 (wherein R^{9'} denotes a hydrogen atom, acetoxy group, trimethylsilyloxy group, triethylsilyloxy group, methoxymethyloxy group or benzyloxymethyloxy group); and
A denotes a methyl group.

11. The 10-oxocyclovitamin D derivative denoted by formula (VI): 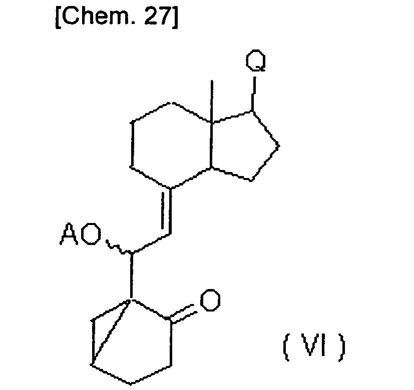 (wherein Q denotes a hydrogen atom, optionally substituted C₁ to C₅ alkyl group, or formula (Q-I): 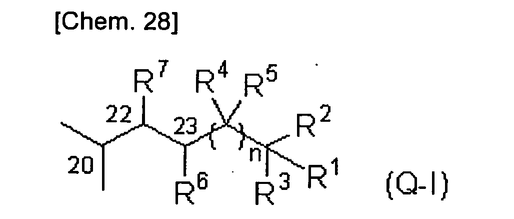 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom);
and A denotes a C₁ to C₃ alkyl group, excluding from the above combinations the case where R¹ denotes a hydroxyl group, R² and R³ denote methyl groups, R⁴, R⁵, R⁶, and R⁷ denote hydrogen atoms, n = 1, and
A denotes a methyl group).

12. The 10-oxocyclovitamin D derivative of Claim 11, wherein Q denotes formula (Q-I): 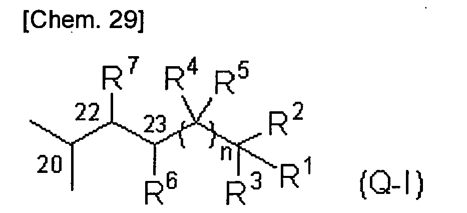 (wherein R¹ denotes a hydrogen atom, hydroxyl group or -OR^{1'}, where R^{1'} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group, or propyl group; each of R⁴ and R⁵ denotes a hydrogen atom or methyl group; each of R⁶ and R⁷ denotes a hydrogen atom, or R⁶ and R⁷ combine to form a carbon-carbon double bond; n denotes an integer of 1 to 3; one of the carbons at position 20, 22 or 23 is optionally substituted with an oxygen, sulfur or nitrogen atom, excluding from the above combinations the case where R¹ denotes a hydroxyl group, R² and R³ denote methyl groups, and R⁴, R⁵, R⁶, and R⁷ denote hydrogen atoms, n = 1); and
A denotes a methyl group.

13. The 10-oxocyclovitamin D derivative of Claim 11, wherein Q denotes formula (Q-II): 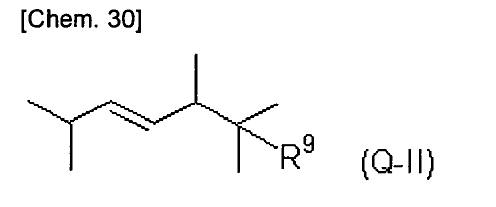 (wherein R⁹ denotes a hydrogen atom, acetoxy group, hydroxyl group, trimethylsilyloxy group, triethylsilyloxy group, methoxymethyloxy group or benzyloxymethyloxy group)
or formula (Q-III): 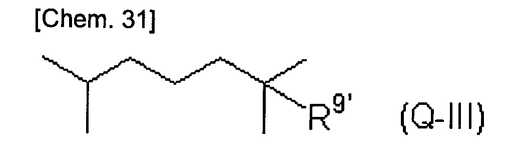 (wherein R^{9'} denotes a hydrogen atom, acetoxy group, trimethylsilyloxy group, triethylsilyloxy group, methoxymethyloxy group or benzyloxymethyloxy group); and
A denotes a methyl group.

14. The cyclovitamin D derivative denoted by formula (VII): 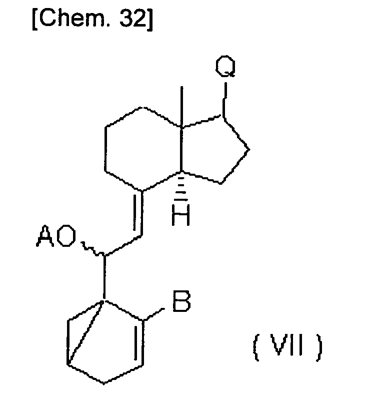 (wherein Q denotes a hydrogen atom, optionally substituted C₁ to C₅ alkyl group, or formula (Q-I): 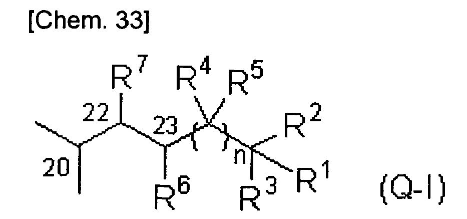 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22, or 23 is optionally substituted with an oxygen, sulfur or nitrogen atom);
A denotes a C₁ to C₃ alkyl group; and
B denotes a halogen atom or R⁸SO₂O- (where R⁸ denotes an optionally substituted C₁ to C₆ alkyl group, optionally substituted C₂ to C₆ unsaturated alkyl group, optionally substituted C₆ to C₁₄ aryl group, or optionally substituted C₇ to C₂₂ aralkyl group).

15. The cyclovitamin D derivative according to Claim 14, wherein Q denotes formula (Q-I): 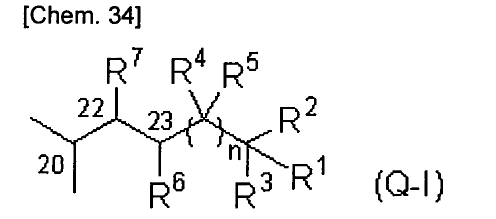 (wherein R¹ denotes a hydrogen atom, hydroxyl group or -OR^{1'}, where R^{1'} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group or propyl group; each of R⁴ and R⁵ denotes a hydrogen atom or methyl group; each of R⁶ and R⁷ denotes a hydrogen atom, or R⁶ and R⁷ combine to form a carbon-carbon double bond; n denotes an integer of 1 to 3; and one of the carbons at position 20, 22 or 23 is optionally substituted with an oxygen, sulfur or nitrogen atom);
A denotes a methyl group; and
B denotes R⁸SO₂O- (where R⁸ denotes an optionally substituted C₁ to C₄ alkyl group, optionally substituted C₆ or C₇ aryl group, optionally substituted C₇ or C₈ aralkyl group).

16. The cyclovitamin D derivative according to Claim 14, wherein Q denotes formula (Q-II): 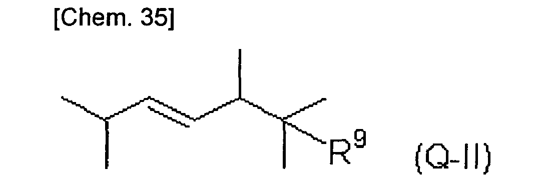 (wherein R⁹ denotes a hydrogen atom, acetoxy group, hydroxyl group, trimethylsilyloxy group, triethylsilyloxy group, methoxymethyloxy group or benzyloxymethyloxy group)
or formula (Q-III): 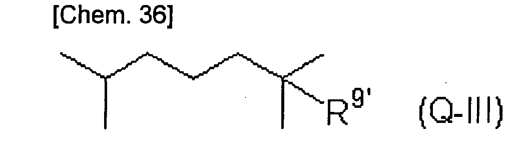 (wherein R^{9'} denotes a hydrogen atom, acetoxy group, trimethylsilyloxy group, triethylsilyloxy group, methoxymethyloxy group or benzyloxymethyloxy group); A denotes a methyl group; and
B denotes a trifluoromethanesulfonyloxy group.

17. The 1,10-olefincyclovitamin D derivative denoted by formula (VIII): 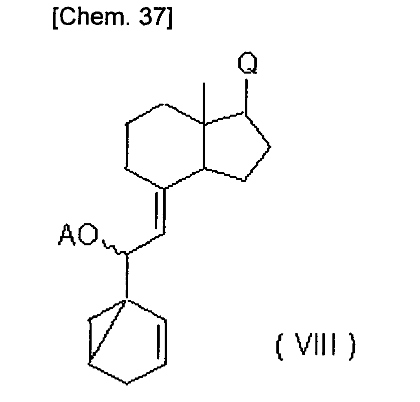 (wherein Q denotes a hydrogen atom, optionally substituted C₁ to C₅ alkyl group, or formula (Q-I): 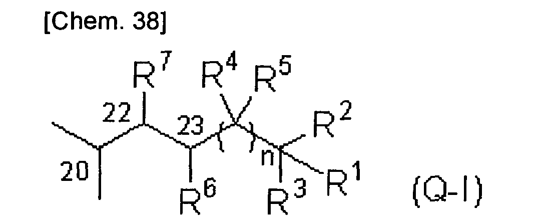 (wherein R¹ denotes a hydrogen atom, hydroxyl group or -OR^{1'}, where R^{1'} denotes an optionally substituted C₂ to C₄ acyl group, optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form - (CH₂)m- (where m denotes an integer of 2 to 5); each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, optionally substituted C₂ to C₇ acyloxy group, or R⁶ and R⁷ combine to form a carbon-carbon double bond; n denotes an integer of 1 to 5; and one of the carbons at position 20, 22 or 23 is optionally substituted with an oxygen, sulfur or nitrogen atom); and
A denotes a C₁ to C₃ alkyl group).

18. The 1,10-olefincyclovitamin D derivative according to Claim 17, wherein Q denotes formula (Q-I): 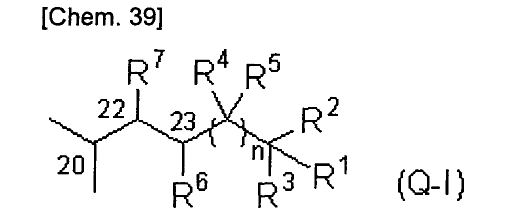 (wherein R¹ denotes a hydrogen atom, hydroxyl group or -OR^{1'}, where R^{1'} denotes a C₂ or C₃ acyl group, trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group, or propyl group; each of R⁴ and R⁵ denotes a hydrogen atom or methyl group; each of R⁶ and R⁷ denotes a hydrogen atom or R⁶ and R⁷ combine to form a carbon-carbon double bond; n denotes an integer of 1 to 3; and one of the carbons at position 20, 22 or 23 is optionally substituted with an oxygen, sulfur or nitrogen atom); and
A denotes a methyl group.

19. The 1,10-olefincyclovitamin D derivative according to Claim 17, wherein Q denotes formula (Q-II): 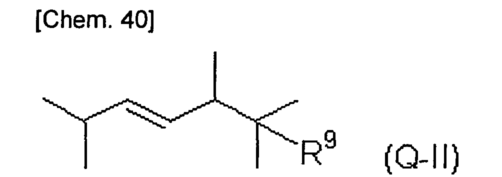 (wherein R⁹ denotes a hydrogen atom, acetoxy group, hydroxyl group, trimethylsilyloxy group, triethylsilyloxy group, methoxymethyloxy group or benzyloxymethyloxy group)
or formula (Q-III): 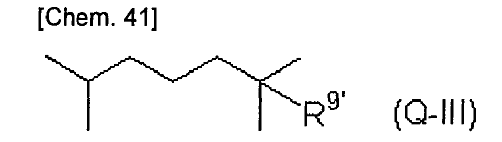 (wherein R^{9'} denotes a hydrogen atom, acetoxy group, trimethylsilyloxy group, triethylsilyloxy group, methoxymethyloxy group or benzyloxymethyloxy group); and A denotes a methyl group.

20. The 1-hydroxy-19-norcyclovitamin D derivative denoted by formula (IX): 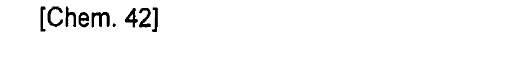 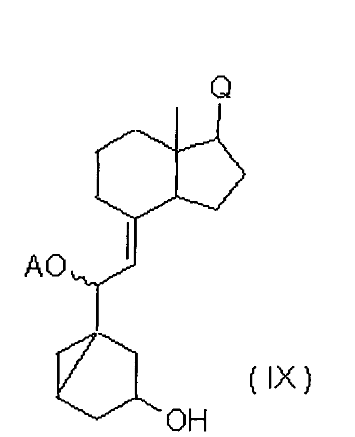 (wherein Q denotes formula (Q-IV): 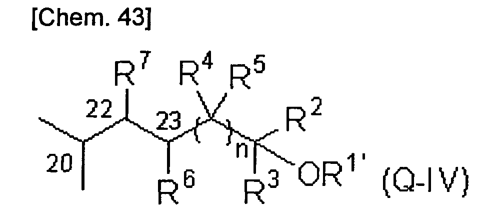 (wherein R^{1'} denotes an optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form -(CH₂)m- (where m denotes an integer of 2 to 5);
each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, or optionally substituted C₂ to C₇ acyloxy group, or R⁶ and R⁷ combine to form a carbon-carbon double bond; n denotes an integer of 1 to 5; and one of the carbon atoms at position 20, 22 or 23 is optionally substituted with an oxygen, sulfur or nitrogen atom); and A denotes a C₁ to C₃ alkyl group.

21. The 1-hydroxy-19-norcyclovitamin D derivative according to Claim 20, wherein Q denotes formula (Q-IV): 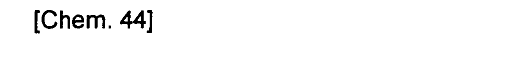 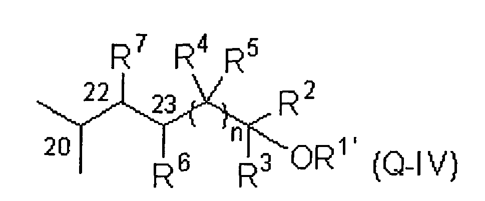 (wherein R^{1'} denotes a trimethylsilyl group, triethylsilyl group, methoxymethyl group, ethoxymethyl group, or optionally substituted C₈ or C₉ aralkyloxyalkyl group; each of R² and R³ denotes a methyl group, trifluoromethyl group, ethyl group, or propyl group; each of R⁴ and R⁵ denotes a hydrogen atom or methyl group; each of R⁶ and R⁷ denotes a hydrogen atom, or R⁶ and R⁷ combine to form a carbon-carbon double bond; n denotes an integer of 1 to 3; and one of the carbon atoms at position 20, 22 or 23 is optionally substituted with an oxygen, sulfur or nitrogen atom); and
A denotes a methyl group.

22. The 1-hydroxy-19-norvitamin D derivative according to Claim 20, wherein Q denotes formula (Q-II): 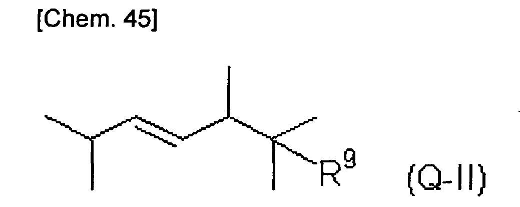 (wherein R⁹ denotes a trimethylsilyloxy group, triethylsilyloxy group, methoxymethyloxy group, or benzyloxymethyloxy group)
or formula (Q-III): 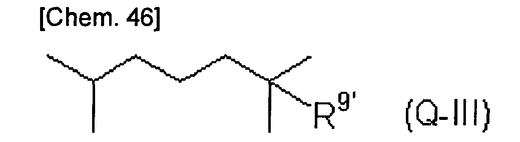 (wherein R^{9'} denotes a trimethylsilyloxy group, triethylsilyloxy group, methoxymethyloxy group or benzyloxymethyloxy group); and
A denotes a methyl group.

23. The 1-hydroxy-19-norvitamin D derivative denoted by formula (X): 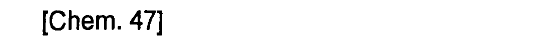 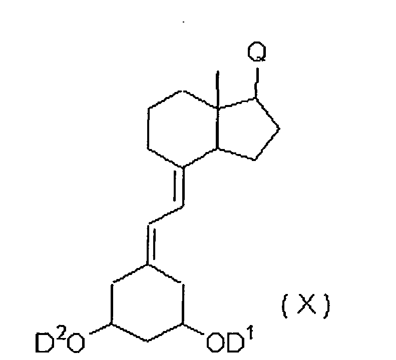 (wherein Q denotes formula (Q-IV): 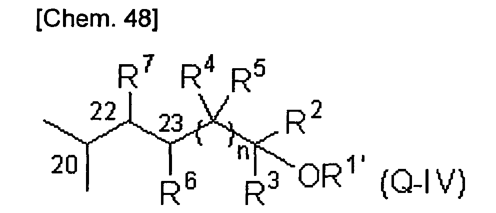 (wherein R^{1'} denotes an optionally substituted C₃ to C₆ alkylsilyl group, optionally substituted C₂ to C₄ alkoxyalkyl group or optionally substituted C₈ to C₁₅ aralkyloxyalkyl group; each of R² and R³ denotes an optionally substituted C₁ to C₄ alkyl group, or R² and R³ combine to form -(CH₂)m- (where m denotes an integer of 2 to 5);
each of R⁴ and R⁵ denotes a hydrogen atom, hydroxyl group, halogen atom, optionally substituted C₂ to C₇ acyloxy group, optionally substituted C₁ to C₆ alkyl group, or R⁴ and R⁵ combine to form a double-bonded oxygen atom; each of R⁶ and R⁷ denotes a hydrogen atom, hydroxyl group, halogen atom, optionally substituted C₁ to C₆ alkyl group, or optionally substituted C₂ to C₇ acyloxy group, or R⁶ and R⁷ combine to form a carbon-carbon double bond; n denotes an integer of 1 to 5; and one of the carbon atoms at position 20, 22 or 23 is optionally substituted with an oxygen, sulfur or nitrogen atom); and each of D¹ and D² denote hydrogen atoms or C₂ to C₇ acyloxy groups.)

24. The 1-hydroxy-19-norvitamin D derivative according to Claim 23, wherein Q denotes formula (Q-IV): 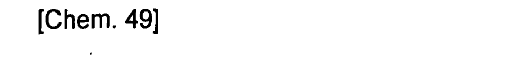 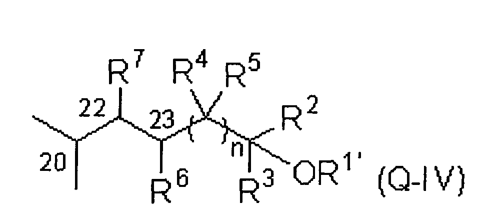 (wherein R^{1'} denotes a trimethylsilyl group, triethylsilyl group, methoxymethyl group, ethoxymethyl group or optionally substituted C₈ or C₉ aralkyloxyalkyl group; each of R² and R³ denotes a methyl group, trifluoromethyl group, ethyl group, or propyl group; each of R⁴ and R⁵ denotes a hydrogen atom or methyl group; each of R⁶ and R⁷ denotes a hydrogen atom or R⁶ and R⁷ combine to form a carbon-carbon double bond; n denotes an integer of 1 to 3; and one of the carbon atoms at position 20, 22 or 23 is optionally substituted with an oxygen, sulfur or nitrogen atom); and
D¹ and D² denote hydrogen atoms or acetyl groups.

25. The 1-hydroxy-19-norvitamin D derivative according to Claim 23, wherein Q denotes formula (Q-II): 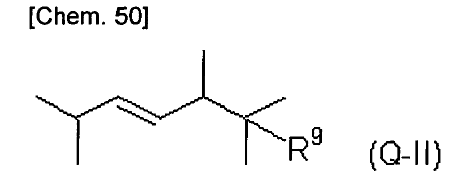 (wherein R⁹ denotes a trimethylsilyloxy group, triethylsilyloxy group, methoxymethyloxy group or benzyloxymethyloxy group)
or formula (Q-III): 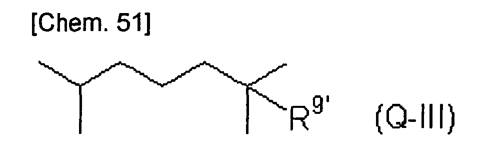 (wherein R^{9'} denotes a trimethylsilyloxy group, triethylsilyloxy group, methoxymethyloxy group or benzyloxymethyloxy group); and
D¹ and D² denotes hydrogen atoms or acetyl groups.
